(19) Europäisches Patentamt
European Patent Office
Office européen des brevets

(11) EP 2 614 281 B1

(12) # EUROPÄISCHE PATENTSCHRIFT

(45) Veröffentlichungstag und Bekanntmachung des Hinweises auf die Patenterteilung:
**14.01.2015 Patentblatt 2015/03**

(21) Anmeldenummer: **11758390.6**

(22) Anmeldetag: **06.09.2011**

(51) Int Cl.:
***F16K 1/44*** (2006.01) ***G01B 7/16*** (2006.01)
***G01N 33/00*** (2006.01) ***G01B 5/30*** (2006.01)
***F16K 37/00*** (2006.01)

(86) Internationale Anmeldenummer:
**PCT/EP2011/004488**

(87) Internationale Veröffentlichungsnummer:
**WO 2012/031743 (15.03.2012 Gazette 2012/11)**

(54) **DIAGNOSE-VERFAHREN FÜR HUBVENTILE UND MESSEINRICHTUNG ZU SEINER DURCHFÜHRUNG**

DIAGNOSTIC METHOD FOR POPPET VALVES AND MEASURING DEVICE FOR CARRYING OUT SAID METHOD

PROCÉDÉ DE DIAGNOSTIC D'UNE SOUPAPE ET DISPOSITIF DE MESURE PERMETTANT LA MISE EN OEUVRE DUDIT PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.09.2010 DE 102010044891**

(43) Veröffentlichungstag der Anmeldung:
**17.07.2013 Patentblatt 2013/29**

(73) Patentinhaber: **GEA Tuchenhagen GmbH**
**21514 Büchen (DE)**

(72) Erfinder:
- **SUEDEL, Matthias**
**23626 Ratekau (DE)**
- **FAHRENBACH, Peter**
**21035 Hamburg (DE)**
- **PORATH, Bernd**
**23881 Breitenfelde (DE)**

(74) Vertreter: **Hauck Patent- und Rechtsanwälte**
**Neuer Wall 50**
**20354 Hamburg (DE)**

(56) Entgegenhaltungen:
**EP-A1- 0 174 384**
**WO-A1-99/60369**
**DE-U1- 29 811 115**
**US-A- 4 882 937**
**US-B1- 6 382 226**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 614 281 B1

Printed by Jouve, 75001 PARIS (FR)

## Beschreibung

TECHNISCHES GEBIET

Die Erfindung betrifft ein Diagnose-Verfahren für Hubventile, mit dem eine von einem Antrieb des Hubventils generierte, eine Aktionskraft darstellende Stellkraft für wenigstens ein Schließglied des Hubventils in Form eines Kraft-Zeit-Verlaufs ermittelt wird, die Ermittlung der Stellkraft entweder unmittelbar oder an einer aus der Stellkraft im Hubventil resultierenden Reaktionskraft vorgenommen wird, wobei die Ermittlung der Stellkraft oder der Reaktionskraft durch eine Messung der von dieser jeweils bewirkten dehnenden Verformungen erfolgt und der gemessene, aktuelle Kraft-Zeit-Verlauf der Stellkraft oder der Reaktionskraft gespeichert und einer Auswertung unterzogen wird. Bei den Hubventilen handelt es sich insbesondere um Absperr-, Doppeldicht-, Doppelsitz- oder sitzreinigungsfähige Doppelsitzventile. Als Antrieb dieser Ventile fungiert bevorzugt ein einseitig mit Druckmittel, vorzugsweise Druckluft, beaufschlagbarer Feder-Kolbenantrieb. Derartige Antriebe arbeiten, bezogen auf das Schließglied und seine Sitzfläche (Absperrventil, Doppeldichtventil) oder auf beide Schließglieder und ihre jeweils zugeordnete Sitzfläche (Doppelsitzventil), federschließend oder federtiffnend. Antriebe mit einem doppeltwirkenden Kolben, der beidseitig mit Druckmittel beaufschlagbar ist, sollen gleichfalls mitumfasst sein.

Bei dem Absperrventil, das als Hubventil mit einem einzigen Schließglied ausgebildet ist (nachfolgend wird diese Ausführung auf die Bezeichnung Absperrventil reduziert), wirkt eine am Schließglied angeordnete Sitzdichtung in Hubrichtung auf die zugeordnete Sitzfläche. Letztere kann senkrecht zur Hubrichtung orientiert sein. In diesem Falle spricht man von axial wirkender Sitzdichtung (Dichtung im Druckeingriff). Die Sitzfläche kann aber auch konisch ausgeführt sein, sodass die von der Sitzfläche auf die Sitzdichtung wirkende Reaktionskraft eine axial und eine radial wirkende Dichtungskraft generiert (Dichtung im Druck- und Gleiteingriff). Das Schließglied kann aber auch als Schieberkolben ausgeführt sein, dessen Sitzdichtung auf der Mantelfläche des Schieberkolbens angeordnet ist und unter radialer Vorspannung auf einer zylindrischen Sitzfläche entlanggleitet (Dichtung im Gleiteingriff).

Die vorstehenden Dichtungskonfigurationen sind gleichermaßen auf Doppeldicht- und (sitzreinigungsfähige) Doppelsitzventile übertragbar. Im Unterschied zum vorg. Absperrventil soll unter einem Doppeldichtventil ein weiteres Absperrventil verstanden werden, das ein einziges Schließglied mit zwei axial, d.h. in Hubrichtung voneinander beabstandeten Sitzdichtungen aufweist, die die zwischen sich und in Verbindung mit zugeordneten Sitzflächen und dem Schließglied einen Leckagehohlraum einschließen, der über mindestens einen Verbindungsweg mit der Umgebung des Doppeldichtventils verbunden ist.

Ein (sitzreinigungsfähiges) Doppelsitzventil besitzt zwei unabhängig voneinander betätigbare Schließglieder, die zwischen sich einen Leckagehohlraum einschließen, der über mindestens einen Verbindungsweg mit der Umgebung des Doppelsitzventils verbunden ist. Jedes Schließglied verfügt über eine zugeordnete Sitzfläche. Bei der Öffnungsbewegung öffnet zunächst das unabhängig angetriebene Schließglied, welches bei seiner weiteren Öffnungsbewegung am anderen Schließglied zur Anlage gelangt und dieses gleichfalls in die Offenstellung überführt. Beim Schließvorgang kehrt sich die vorg. Abfolge der Schritte sinngemäß um. Doppelsitzventile unterscheiden sich von jenen, die sitzreinigungsfähig sind, allein dadurch, dass der zugeordnete Antrieb befähigt ist, die beiden Schließglieder jeweils in getrennt voneinander ansteuerbare Teiloffenstellungen zu überführen.

Das Schließglied ist über eine zugordnete Verstellstange mit dem Antriebskolben des vorstehend kurz skizzierten Antriebs verbunden. Beim Doppelsitzventil besitzt nur das unabhängig angetriebene Schließglied eine feste Verbindung mit dem Antriebskolben im Antrieb, während das abhängig angetriebene Schließglied relativ beweglich zu dem unabhängig angetriebenen Schließglied angeordnet ist und sich unter Federvorspannung gegenüber diesem abstützt. Hinsichtlich der Schließglied- und Dichtungskonfiguration werden heute bevorzugt Doppelsitzventile mit zwei als Sitzteller arbeitenden Schließgliedern (Doppelsitzventil erster Art) oder mit einem als Sitzteller und einem als Schieberkolben ausgebildeten Schließglied eingesetzt, wobei der Schieberkolben das unabhängig angetriebene Schließglied darstellt (Doppelsitzventil zweiter Art).

STAND DER TECHNIK

Der Zustand eines Hubventils hinsichtlich unter anderem

- seines Reibungsverhaltens in den Gehäusedurchführungen seiner Verstellstange(n),
- seiner an dem Schließglied oder den Schließgliedern angreifenden Druck- und Strömungskräfte,
- seines Zustandes der Sitzdichtung(en),
- seines Antriebs (Intaktheit u.a. der Feder, des Antriebskolbens) und
- seiner sonstigen Zustandsparameter

bildet sich insbesondere beim Schalten in der Stellkraft (Aktionskraft) der Verstellstange(n) ab.

Die Druck- und Strömungskräfte aus statischem Druck (Überdruck, Unterdruck) und/oder dynamischem Druck (Anströmung) umfassen die planmäßigen Kräfte, die sich aus einem regulären Betriebsablauf eine Prozessanlage ergeben, in der das Hubventil angeordnet ist, sie umfassen aber auch außerplanmäßige Kräfte, wie beispielsweise Druckschläge oder -stöße im vom jeweiligen Fluid beaufschlagten Ventilgehäuse. Diese außerplanmäßigen Kräfte können die planmäßigen um ein Vielfaches übertreffen, sie greifen am in Frage kommenden Schließglied und damit an der mit diesem fest verbundenden Verstellstange an und haben, sofern sie nicht durch sog. Druckausgleichskolben weitgehend kompensiert sind oder über die Sitzfläche des Schließglieds entsprechende Gegenkräfte erfahren, somit auch Einfluss auf das Kräftespiel und -gleichgewicht im Antrieb.

In der DE 298 11 115 U1 ist ein Messsystem zur Erfassung der Spindelkraft an Armaturen beschrieben, bei dem unter anderem

- ein Kraftsensor kraftschlüssig an einer Position in Kraftrichtung der wirkenden Spindelkraft angeordnet ist,
- der Kraftsensor ein Dehnmessstreifen (DMS) sein kann,
- der Kraftsensor beispielsweise an der Spindel oder an einer Verbindungsschraube an einem Flansch zwischen Armatur und Antrieb montiert ist.

Das bekannte Messsystem weist zusätzlich zu dem als Kraftsensor fungierenden Dehnmessstreifen eine Kalibriermesseinrichtung auf, mit der der Kraftsensor kalibriert wird. Die Kalibriermesseinrichtung wird während des Anlagenbetriebs entfernt. Das beschriebene Messsystem hat sich zur Aufgabe gestellt, ein Messsystem bereitzustellen, welches für das Messverfahren aus der WO 96/30684 A1**,** bei welchem die Spindelkraft in situ gemessen und unter Anwendung von Verfahren der Analyse des Zeitsignals ausgewertet wird, optimal geeignet ist und darüber hinaus eine kontinuierliche Erfassung der Spindelkraft zu ermöglichen.

Ein konkreter Lösungsweg, wie man zur Messung der Spindelkraft eines über ein Spindel/Mutter-System angetriebenes Ventils gelangt, ist nur in Verbindung mit einer Kalibriermesseinrichtung offenbart. Darüber hinaus wird lediglich ein Kraftsensor beschrieben, der an einer Verbindungsschraube an einem Flansch zwischen Armatur und Antrieb montiert sein kann. Ob eine derartige Anordnung geeignet ist, um beispielsweise einen Defekt der Sitzdichtung, eine ausgeschlagene oder "fressende" Stangendurchführung, eine nicht vollzogene Schließ-, Offen- oder Teiloffenstellung (Sitzreinigung) oder einen Druckschlag sicher zu detektieren, wird nicht offenbart, thematisiert oder nahegelegt.

Aus der US 4 882 937 A ist ein Diagnoseverfahren bekannt, das sich auf einen Kraft-Bewegungs-Verlauf stützt. Dabei induziert die Bewegung der Ventilstange in einem Sensor, der im Wesentlichen aus einer Spule mit einer Vielzahl von Windungen besteht, eine Spannung, die proportional zur Geschwindigkeit der axialen Bewegung der Ventilstange sein soll. Es wird somit ein Geschwindigkeits-Zeit-Verlauf ermittelt, der mit dem gleichfalls gemessenen Kraft-Zeit-Verlauf verknüpft wird. Dabei wird nicht die Stellkraft der Ventilstange direkt, sondern deren Reaktionskraft, die sich in einem Verbindungsgehäuse zwischen einem Ventilgehäuse und einem die Stellbewegung der Ventilstange bewirkenden Antrieb abbildet, über einen geeigneten Sensor gemessen.

Konkrete Lösungswege, wie man zu einem praktikablen Diagnose-Verfahren für Hubventile mit einem druckmittelbeaufschlagten Feder-Kolben Antrieb und einer Messeinrichtung zu seiner Durchführung gelangt, sind somit im vorgenannten Stand der Technik nicht offenbart. Dieser Stand der Technik offenbart auch keine Hinweise und Anregungen, wie sich aus den gewonnenen Messsignalen diesbezügliche Erkenntnisse gewinnen lassen

Ausgehend von diesem Stand der Technik ist es die Aufgabe der vorliegenden Erfindung, ein Diagnose-Verfahren für Hubventile der gattungsgemäßen Art anzugeben, mit dem der Zustand eines Hubventils hinsichtlich u.a.

- des Reibungsverhaltens in den Durchführungen seiner Verstellstange(n)
- des Zustandes seiner Sitzdichtung(en)
- mechanischer Beschädigungen seiner miteinander im Wechselspiel stehender Bauteile (z.B. Feder(n) im Antrieb; Federbruch)
- der Hublage seines Schließgliedes/seiner Schließglieder
  im Zuge seiner Schaltzyklen und/oder
- Ereignisse, wie das Auftreten von Druckschlägen oder -stößen im laufenden Betrieb

lückenlos überwacht wird/werden, die Messsignale gespeichert und die gespeicherten Messsignale zielführend interpretiert werden und eine Frühanzeige von Schadensfällen durchgeführt wird. Darüber hinaus ist es weiterhin Aufgabe der Erfindung, eine Messeinrichtung zur Durchführung des Diagnose-Verfahrens anzugeben, mit der die vorgenannten Zustände und Ereignisse sicher und reproduzierbar zu detektieren sind.

TECHNOLOGISCHER HINTERGRUND

Bevor die Erfindung näher erläutert wird, sollen nachfolgend zunächst die grundlegenden Kräfteverhältnisse

beim Schalten eines Absperrventils und eines Doppelsitzventils dargestellt werden, die es erlauben, den für den Zustand eines Hubventils maßgeblichen Kraft-Zeit-Verlauf bzw. den Kraft-Hub-Verlauf in Form der als Aktionskraft fungierenden Stellkraft oder in Form einer von der Stellkraft im Hubventil generierten Reaktionskraft zu detektieren. Die Kräfteverhältnisse werden beispielhaft für den Vorgang des Schließens an einem Hubventil 100, und zwar an einem federschließenden Absperrventil 110 **(Figur 1),** und an einem federschließenden Doppelsitzventil 120 **(Figur 2)** dargestellt.

**Absperrventil (Figur 1)**

**Figur 1** der Zeichnung verdeutlicht das Kräftespiel im Zuge eines Schaltzyklus eines Absperrventils 110, wobei nachfolgend der Schließvorganges bis zur Schließstellung SS (Hubbewegung erfolgt in (-)y-Richtung) aus diesem Schaltzyklus betrachtet wird. In der Schließstellung SS wirkt eine Sitzdichtung 16* mit einer Dichtungskraft F5 in (+)y-Richtung auf ein einziges Schließglied 8* und damit auf eine Verstellstange 8a* ein. Während des beispielhaft ausgewählten Schließvorganges wirken weiterhin die Reibungskräfte F3 in der Durchführung der Verstellstange 8a* durch einen zweiten Ventilgehäuseteil 1b eines Ventilgehäuses 1, und zwar im Führungsring (nicht bezeichneter oberer Ring) und in der Stangendichtung (nicht bezeichneter unterer Ring) in (+)y-Richtung. Weiterhin wirken ggf. in dem zweiten und in einem ersten Ventilgehäuseteil 1b, 1a Strömungskräfte und/oder Druckkräfte F4 auf das Schließglied 8* in (+)y- oder (-)y-Richtung (statischer Druck, auch Besaugung bei Unterdruck, dynamischer Druck, Gesamtdruck, Druckschlag). Damit ergibt sich für eine an der Verstellstange 8a* angreifende Stellkraft F1 aus dem Kräftegleichgewicht an der Verstellstange 8a*, unterhalb des oberen Endes eines Laternengehäuses 4 (positive Kraftrichtung +y nach oben):

$$- F1 + F3 +/- F4 + F5 = 0 \qquad (1)$$

$$F1 = F3 +/- F4 + F5. \qquad (1a)$$

Die Stellkraft F1 der Ventilstange 8a* (Aktionskraft; Gleichung (1a)) ist beim Schließvorgang im Regelfall (Stangenreibung F3 in (+)y-Richtung wirkend), bezogen auf die Verstellstange 8a*, eine Druckkraft. Dabei bleibt bei diesem Ergebnis der Einfluss der Strömungskraft F4 unberücksichtigt.

Beim Öffnungsvorgang kehren sich die jeweiligen Kraftrichtungen entsprechend um, bis auf die auf das Schließglied 8* wirkende Dichtungskraft F5, die stets so lange in (+)y-Richtung wirkt, wie die Sitzdichtung 16* gegen eine zugeordnete Sitzfläche 12* gepresst wird. Dabei bleibt bei diesem Ergebnis gleichfalls der Einfluss der Strömungskraft F4 unberücksichtigt.

Die Stellkraft F1 als Aktionskraft zwischen einem Antrieb 2 und dem Ventilgehäuse 1, insbesondere im Zusammenwirken des Schließglieds 8* mit der Sitzfläche 12*, und zwar beim Einfahren des Schließgliedes 8* in die oder Ausfahren aus der Sitzfläche 12*, findet ihre Gegenkraft, eine Reaktionskraft F2, in besonders überschaubarer Ausprägung in dem Laternengehäuse 4, das eine feste Verbindung zwischen dem Ventilgehäuse 1 und dem Antrieb 2 herstellt, wobei das Laternengehäuse 4 in einer bevorzugten Ausgestaltung aus zwei diametral zueinander angeordneten Verbindungsstegen, zwei Laternentraversen 4a, 4b, besteht. Jede Änderung der Aktionskraft F1 unterhalb des Antriebs 2, d.h. unterhalb des oberen Endes des Latemengehäuses 4, findet im Laternengehäuse 4 ihre jeweilige entgegengerichtete Reaktionskraft F2. Für das Kräftegleichgewicht (Gleichungen (2), (2a)) am vom Laternengehäuse 4 gehaltenen Ventilgehäuse 1 gilt ((+)y-Richtung):

$$- F1 + F2 = 0 \qquad (2)$$

$$F1 = F2 \qquad (2a)$$

An der Verstellstange 8a* innerhalb des Antriebs 2 angreifende Kräfte generieren keine Gegenkräfte im Laternengehäuse 4; sie werden im Antrieb 2 vollständig ausgeglichen. Beim Schließvorgang wirken Druckkräfte auf die Verstellstange 8* und Zugkräfte auf das Laternengehäuse 4. Beim Öffnungsvorgang kehren sich die Beanspruchungsrichtungen entsprechend um, sobald keine Dichtungspressung mehr vorliegt und das Schließglied 8* nicht mit der die Reibungskraft F3 in (+)y-Richtung überwiegenden Strömungskraft F4 beaufschlagt wird.

Es gilt weiterhin:

- In der Schließstellung SS des Absperrventils 110 stützt sich das Schließglied 8* mit einer im Antrieb 2 vorgesehenen minimalen Vorspannkraft FVo einer Feder 2.5 **(Figuren 1a**, **1b)** auf der Sitzdichtung 16* und damit auf der Sitzfläche 12* ab. Diese Aktions- und Stellkraft F1 äußert sich als Druckkraft in der Verstellstange 8a*. Sie generiert im Laternengehäuse 4 die Reaktionskraft F2 in Gestalt einer gleichgroßen Zugkraft (zwecks Herstellung des Kräftegleichgewichts am Absperrventil 110 unterhalb des Antriebs 2).

- Wird das Absperrventil 110 nun geöffnet, dann bauen sich auf dem relativ kurzen Weg der Entspannung der Sitzdichtung 16* diese Kräfte im Sitzbereich ab. Falls nach dem Verlassen der Sitzfläche 12* keine Kräfte mehr auf das Schließglied 8* wirken - diese Vereinfachung an dieser Stelle nur, um die Konsequenzen zu verdeutlichen - dann ist das Laternengehäuse 4 frei von Zug- oder Druckkräften. Das notwendige Kräftegleichgewicht des angesteuerten und nunmehr unter einer Vorspannkraft FV der Feder 2.5 stehenden Antriebs 2 **(Figuren 1a, 1b)**, die größer als die ursprüngliche minimale Vorspannkraft FVo in der Schließstellung SS ist, wird innerhalb des Antriebs 2 hergestellt.

- Wirken auf die Verstellstange 8a* zusätzlich Reibungskräfte F3, z.B. an der Durchführung der Verstellstange 8a* durch das Ventilgehäuse 1, oder auf das Schließglied 8* zusätzlich Strömungskräfte F4 und damit wiederum auf die Verstellstange 8a*, dann bilden sich diese Kräfte ebenso jeweils zwangsläufig als Reaktionskräfte F2 in dem Laternengehäuse 4 ab.

- Die signifikanteste Kraftbeaufschlagung des Latemengehäuses 4 erfolgt beim Einfahren des Schließgliedes 8* mit seiner Sitzdichtung 16* in die und beim Ausfahren aus der Sitzfläche 12*, wobei maximal die minimale Vorspannkraft FVo der Feder 2.5 in der Schließstellung SS des Absperrventils 110 im Laternengehäuse 4 generiert wird. Andere vorstehend genannten Kräfte überlagern sich ggf. diesem Kräftespiel.

<u>**Doppelsitzventil (Figur 2)**</u>

Bei einem als Doppelsitzventil 120, 130 gemäß **Figur 2** ausgebildeten Hubventil 100, wie es hinsichtlich seiner Schließglied- und Sitzkonfiguration und der daraus resultierenden Bewegungskinematik aus der EP 1 529 176 B1 bekannt ist (Doppelsitzventil erster Art), oder bei einem Doppelsitzventil zweiter Art mit einem als Schieberkolben ausgebildeten, unabhängig angetriebenen ersten Schließglied und einem als Sitzteller ausgebildeten, abhängig angetriebenen zweiten Schließglied (DE 196 08 792 C2), gelten sinngemäß die gleichen Überlegungen hinsichtlich des Kräftespiels und des -gleichgewichts wie bei dem vorstehend erörterten Absperrventil 110. Die beiden Doppelsitzventil-Typen unterscheiden sich allerdings signifikant in ihrem Stellkraft- und Reaktionskraft-Verlauf beim Einfahren der Schließglieder in die und beim Ausfahren aus den zugeordneten Sitzflächen. Diese Unterschiede werden unter den diesbezüglichen Messergebnissen weiter unten aufgezeigt.

Das Doppelsitzventil 120, 130 (Figur 2) weist ein von einem federschließenden Antrieb 2 unabhängig angetriebenes erstes Schließglied 6 und ein von letzterem abhängig angetriebenes zweites Schließglied 8 auf, die zwischen sich einen Leckagehohlraum 7 einschließen, wobei sich beispielsweise eine mit dem zweiten Schließglied 8 fest verbundene, als Hohlstange ausgebildete zweite Verstellstange 8a unter der Vorspannkraft einer zweiten Feder 2.7 an einer mit dem ersten Schließglied 6 fest verbundenen, in der Hohlstange 8a konzentrisch hindurchgeführten ersten Verstellstange 6a abstützt. Während das erste Schließglied 6 in seiner Schließstellung mittels der Feder 2.5 im Antriebs 2 mit einer ersten Sitzdichtung 14 auf eine zugeordnete erste Sitzfläche 10 gedrückt wird, erfolgt die entsprechende Anpressung des zweiten Schließglieds 8 mit einer zweiten Sitzdichtung 16 auf eine zugeordnete zweite Sitzfläche 12 mittels der zweiten Feder 2.7.

Die vorstehenden Darlegungen zum Kräftespiel am Absperrventil 110 sind uneingeschränkt auf jedes Schließglied 6, 8 übertragbar, weil an jedem vergleichbare ursächliche Kräfte angreifen können (F3.1, F3.2; F4.1, F4.2; F5.1, F5.2). Zwischen den beiden Schließgliedern 6, 8 ist eine Mitteldichtung 18 angeordnet, die im Zuge der Öffnungsbewegung des ersten Schließglieds 6, ausgehend von der Schließstellung SS gemäß Figur 2, nach einem Teilhub des ersten Schließglieds 6 am zweiten Schließglied 8 zum Eingriff kommt und in einer nachfolgenden gemeinsamen Offen- (OS) oder Teiloffenstellung im Eingriff bleibt, in der in Figur 2 dargestellten Schließstellung SS jedoch außer Eingriff ist. Die Kopplung und Entkopplung der beiden Schließglieder 6, 8 im Verlauf eines aus Öffnungs- und Schließbewegung bestehenden Schaltzyklus erfolgt demnach unter den Verformungsgegebenheiten der Mitteldichtung 18.

Wie **Figur 2** zeigt, lässt sich für jedes Schließglied 6, 8 eine Kräftebilanz und ein Kräftegleichgewicht entsprechend der Gleichung (1a) mit folgendem Ergebnis aufstellen (Gleichungen (3), (4)): erstes Schließglied 6:

$$F1.1 = F3.1 +/- F4.1 + F5.1. \quad (3)$$

zweites Schließglied 8:

$$F1.2 = F3.2 +/- F4.2 + F5.2. \quad (4)$$

Daraus folgt für das Kräftegleichgewicht an, einer Kolbenstange 2.6 unterhalb des Laternengehäuses 4, wo eine erste Stellkraft F1.1 und eine zweite Stellkraft F1.2 zur Stellkraft F1 zusammengeführt werden:

$$F1 = F1.1 + F1.2 \quad (5)$$

Für die Reaktionskraft F2 im Laternengehäuse 4 gilt mit Gleichung (2a):

$$F1 = F2 = F1.1 + F1.2. \quad (6)$$

Aufgrund der Tatsache, dass jedes Schließglied 6, 8, unabhängig vom anderen, mit seiner Sitzdichtung 14, 16 in die zugeordnete Sitzfläche 10, 12 ein- und aus diesen ausfährt, ist mit dem Ergebnis der Gleichung (6) gezeigt, dass eine selektive Diagnose der Schalt- und Zustandsbedingungen der beiden Schließglieder 6, 8 möglich ist. In dem vorstehenden Diagnose-Ansatz für Doppelsitzventile ist eine eigenständige Erfindung begründet.

## ZUSAMMENFASSUNG DER ERFINDUNG

Die der Erfindung zugrundeliegende Aufgabe wird mit einem Diagnose-Verfahren mit den Merkmalen des Anspruchs 1 gelöst. Vorteilhafte Ausgestaltungen des Verfahrens sind Gegenstand der Unteransprüche. Eine Messeinrichtung zur Durchführung des Diagnoseverfahrens ist Gegenstand des unabhängigen Anspruchs 8. Vorteilhafte Ausführungsformen der Messeinrichtung sind Gegenstand der nachfolgenden Unteransprüche. Die Anwendung des Diagnose-Verfahrens und der Messeinrichtung zu seiner Durchführung auf spezielle Hubventile ist Gegenstand der abhängigen Ansprüche 11 bis 14.

Das Diagnose-Verfahren zur Lösung der gestellten Aufgabe sieht vor, dass zeitlich parallel zum Kraft-Zeit-Verlauf der Stellkraft F1(t) oder der Reaktionskraft F2(t) ein Hub-Zeit-Verlauf h(t) eines Hubs h des wenigstens einen Schließglieds gemessen wird, dass der Kraft-Zeit-Verlauf der Stellkraft oder der Reaktionskraft und der Hub-Zeit-Verlauf miteinander verknüpft werden und daraus ein Kraft-Hub-Verlauf der Stellkraft F1(h) oder der Reaktionskraft F2(h) ermittelt wird. Der in dieser Weise über die Betriebs- oder Lebensdauer des Hubventils jeweils ermittelte aktuelle Kraft-Hub-Verlauf der als Aktionskraft fungierenden Stellkraft F1 (h) oder der Reaktionskraft F2(h) eines Schaltzyklus wird mit einem gespeicherten, früheren verglichen. Aus dem erfindungsgemäßen Vergleich werden Abweichungen ermittelt, die innerhalb eines vorgegebenen Toleranzbereichs für diese Abweichungen akzeptiert werden oder die bei Überschreitung des vorgegebenen Toleranzbereichs durch diese Abweichungen eine Meldung und/oder ein Steuersignal generieren.

Dabei liegt die Erfassungsstelle der Stellkraft nach Maßgabe des vorstehend erörterten Kräftespiels an der zugeordneten Verstellstange, irgendwo zwischen dem Schließglied und dem Austritt der Verstellstange aus dem Antrieb im Bereich des Laternengehäuses. Wird im Diagnose-Verfahren der Kraft-Zeit-Verlauf der Reaktionskraft F2(t) herangezogen, dann liegt die Erfassungsstelle der Reaktionskraft im Umgebungsbereich der Verstellstange und zwischen Antrieb und Ventilgehäuse. Diese Erfassungsstelle liegt, wie dies eine Ausgestaltung der Messeinrichtung zur Durchführung des Diagnose-Verfahrens vorsieht, in vorteilhafter Weise am oder im tragenden Bereich des Laternengehäuses, durch das der Kraftfluss der Reaktionskraft hindurchgeleitet werden muss. Die Erfassungsstelle der Reaktionskraft kann aber auch an einer Verbindungsstelle zwischen Laternengehäuse und Ventilgehäuse oder zwischen Laternengehäuse und Antrieb angeordnet sein, wobei auch die Verbindungsmittel selbst an der Verbindungsstelle als Ort der Wahl in Frage kommen. Die Kraftmessung erfolgt dabei nicht direkt über Kraftsensoren, sondern indirekt durch Messung der durch die Stell- oder Reaktionskraft hervorgerufenen dehnenden Verformung mittels Dehnungssensoren, die beispielsweise in sog. Dehnungsmessstreifen (Abk.: DMS, englisch: strain gauge) ihre bevorzugte Ausprägung finden.

Aus dem erfindungsgemäßen Vergleich werden Abweichungen ermittelt, die innerhalb eines vorgegebenen

Toleranzbereichs für diese Abweichungen akzeptiert werden oder die bei Überschreitung des vorgegebenen Toleranzbereichs durch diese Abweichungen eine Meldung in Form einer Verschleiß-, Wartungs- oder Beschädigungsmeldung (akustische oder optische Ausgabe, Druckausgabe, u.a.) und/oder ein Steuersignal generieren.

Die Hubermittlung in der vorstehend beschriebenen Weise ist konstruktiv aufwendig und erfordert zusätzliche Bauhöhe in Form der vorg. Steuereinheit. Um die Hubermittlung zu vereinfachen, schlägt die Erfindung vor, dass der Hub durch eine diesen innerhalb des Hubventils indirekt abbildende physikalische Größe ermittelt wird, wobei als physikalische Größe eine durch die Erzeugung des Hubs im Antrieb an geeigneter Stelle hervorgerufene Dehnung herangezogen wird. Zu deren Messung können wiederum die vorstehend genannten Dehnungssensoren verwendet werden. Die den Antrieb im Zuge seiner Huberzeugung dehnenden Kräfte resultieren bei einem Feder-Kolben-Antrieb aus der Vorspannkraft der Feder im Antrieb und diese Kräfte werden in dieser Feder adäquat abgebildet. Bei Kenntnis der Federkennlinie - im Regelfalle handelt es sich um eine Hooksche Gerade-können aus der ermittelten dehnenden Kraft wiederum der Federweg und somit schließlich die Hublage des Schließgliedes ermittelt werden.

Die Diagnose des Kraft-Hub-Verlaufs F1(h) oder F2(h) hat gegenüber der Diagnose des Kraft-Zeit-Verlaufs F1(t) oder F2(t) den Vorteil, dass signifikante Ereignisse und Zustandsänderungen unmittelbar der jeweiligen Hublage des Hubventils zugeordnet und so wesentlicher einfacher als bei der zeitlichen Zuordnung zu interpretieren sind. Darüber hinaus gestaltet sich die Auswertung von Kraft-Zeit-Verläufen im Zuge des unmittelbaren Vergleichs zwischen aktuellen und früheren Verläufen schwierig, da besondere Umstände beim Betrieb des Hubventils, die nicht auf Zustandsänderungen des Hubventils, sondern beispielsweise auf Fremdeinflüsse zurückzuführen sind, die Zeitachse des Hub-Zeit-Verlaufs dehnen oder verkürzen können.

Zur Ermittlung des Hub-Zeit-Verlaufs h(t) ist an sich bekannt, den jeweiligen Hub h durch direkte Wegmessung zu ermitteln. Es ist in diesem Zusammenhang bekannt, die aktuelle Stellung eines Hubventils durch einen kontinuierlich arbeitenden Stellungsmelder zu erfassen, der beispielsweise in einer auf der dem Ventilgehäuse abgewandten Seite des Antriebs angeordneten Steuereinheit untergebracht ist. Die Hublage des Schließglieds wird dabei in der Regel über die durch den Antrieb bis in die Steuereinheit hindurchgeführte Verstellstange des Schließglieds abgebildet, wobei der Stellungsmelder die Hublage dieser Verstellstange detektiert und ein dem Hub proportionales Messsignal liefert. Ein diesbezügliches Wegmesssystem ist beispielsweise in der WO 02/093 058 A1 oder der EP 1 387 975 B1 beschrieben.

Um den Vergleich der Schaltzyklen so einfach wie möglich zu gestalten und gleiche Ausgangsbedingungen sicherzustellen, wird vorgeschlagen, dass ein Schaltzyklus in zeitlicher Abfolge wenigstens aus einer Schließstellung, einer Öffnungsbewegung, einer Offen- oder Teiloffenstellung und/oder wenigstens einer Offen- oder Teiloffenstellung, einer Schließbewegung und einer Schließstellung besteht.

Mit Blick auf den Vergleich selbst schlägt die Erfindung drei bevorzugte Varianten vor. Eine erste Variante sieht vor, dass ein zu Beginn der Betriebs- oder Lebensdauer des Hubventils gemessener, akzeptierter Schaltzyklus jeweils zum Vergleich herangezogen wird. Dies kann beispielsweise der Schaltzyklus eines neuwertigen Hubventils oder jener nach einer definierten, klar abgegrenzten Einlaufphase sein. Die zweite Variante schlägt vor, dass der gemessene, aktuelle Schaltzyklus jeweils mit dem zuletzt gemessenen und akzeptierten verglichen wird. Dabei kann zusätzlich vorgesehen sein, dass der erfindungsgemäße Toleranzbereich mit dem zuletzt gemessenen und akzeptierten Schaltzyklus adäquat verschoben wird. Auf diese Weise können Veränderungen des Hubventils (beispielsweise Setzungen oder temperaturbedingte Verformungen), die nicht aus den zu diagnostizierenden Zustandsänderungen des Hubventils herrühren, kompensiert werden und führen nicht zu einer Fehler- oder Wartungsmeldung oder einem unerwünschten Steuersignal.

Die dritte Verfahrensvariante verbessert die Datenbasis der Vergleichsgröße, indem der gemessene, aktuelle Schaltzyklus jeweils mit dem Mittelwert einer vorbestimmten Anzahl von zuletzt gemessenen und akzeptierten Schaltzyklen verglichen wird. Bei diesem Mittelwert kann es sich um den sog. arithmetischen oder den geometrischen Mittelwert handeln. Von Vorteil ist auch die Anwendung des sogenannten "gleitenden" Mittelwerts, bei dem der jeweils zuletzt gemessene Schaltzyklus in die jeweilige Mittelwertbildung aufgenommen und dafür der jeweils älteste Schaltzyklus verworfen wird. Diese Art der Mittelwertbildung wirkt sich ähnlich aus wie die Verschiebung des Toleranzbereichs im Zusammenhang mit der vorstehend beschriebenen zweiten Verfahrensvariante.

Die Erfindung schlägt weiterhin vor, dass im Rahmen der vorstehend beschriebenen Diagnose-Verfahren zum Vergleich der Kraft-Zeit-Verläufe der Stellkraft F1(t) oder der Reaktionskraft F2(t) oder der Kraft-Hub-Verläufe der Stellkraft F1(h) oder der Reaktionskraft(F2(h) als Vergleichskriterium wenigstens die Steigung oder die Krümmung oder der Betrag der Verläufe an jeweils vorbestimmten diskreten Vergleichsstellen und/oder wenigstens die Betragsänderung oder das Flächenintegral der Verläufe in jeweils vorbestimmten diskreten Zeit- oder Wegintervallen $\Delta t$, $\Delta h$ herangezogen werden/wird. Besonders zielführend ist dabei das Flächenintegral unter dem Kraft-Hub-Verlauf F1(h). F2(h) in einem vorgegebenen Hubintervall $\Delta h$, wobei es sich beim Einfahren der Sitzdichtung in die zugeordnete Sitzfläche um die Kompressionsarbeit

$$W12 = \int_{h1}^{h2} F(h)dh$$

entlang des Verformungsweges $\Delta h = h1 - h2$ und beim Ausfahren aus derselben um die Dekompressionsarbeit W21 an der Sitzdichtung entlang des Verformungsweges $\Delta h = h2 - h1$ handelt. Eine intakte Sitzdichtung liefert eine andere Kompressions- oder Dekompressionsarbeit als eine teilweise, halb oder gänzlich herausgerissene Sitzdichtung.

Das erfindungsgemäße Diagnose-Verfahren ist geeignet, eine Bauart des Hubventils, an dem es zur Anwendung kommt, zu erkennen. Dies gelingt dadurch, dass der Kraft-Hub-Verlauf der Stellkraft oder der Reaktionskraft F1(h, F2(h) zu Beginn der Betriebs- oder Lebensdauer des Hubventils zur Erkennung der Bauart des Hubventils herangezogen und das derart typisierte Hubventil anschließend einer Voreinstellung mit Einstell- und/oder Überwachungsdaten unterzogen wird. Wie die nachfolgend noch gezeigten und erläuterten Messverläufe zeigen, besitzt jede Hubventil-Bauart im Zuge eines Schaltzyklus einen signifikanten, reproduzierbaren, unverwechselbaren Kraft-Zeit-Verlauf oder Kraft-Hub-Verlauf, der sich zur Typisierung eignet.

Zur Durchführung des Diagnose-Verfahrens schlägt die Erfindung eine Messeinrichtung vor, die an einem Hubventil angeordnet ist, wobei das Hubventil in einem Ventilgehäuse wenigstens ein Schließglied aufweist, wobei das Ventilgehäuse über ein Laternengehäuse mit einem Antrieb fest verbunden ist, wobei der Antrieb als druckmittelbeaufschlagter Feder-Kolben-Antrieb ausgeführt ist, wobei wenigstens eine vom Antrieb betätigbare Verstellstange für das wenigstens eine Schließglied vorgesehen ist und wobei am Hubventil eine der Messeinrichtung zugeordnete Auswerteeinrichtung angeordnet ist. Dabei hat es sich als zielführend und insbesondere sehr praxistauglich erwiesen, wenn eine von der Stellkraft im Laternengehäuse generierte Reaktionskraft gemessen wird. Hierzu wird erfindungsgemäß vorgeschlagen, dass die Messeinrichtung wenigstens aus einer ersten Messeinrichtung besteht, die von wenigstens einem Dehnungssensor gebildet, an dem Laternengehäuse angeordnet und mit der Auswerteeinheit verbunden ist. Im geometrisch sehr einfachen Laternengehäuse kann sich die Reaktionskraft eindeutig ausprägen und sie ist dort einer Messung sehr einfach zugänglich.

Zur indirekten Messung des Hub-Zeit-Verlaufs h(t) ist weiterhin eine Hub-Messeinrichtung vorgesehen, die an oder in einem Gehäusemantel des Antriebs angeordnet ist, die von wenigstens einem weiteren Dehnungssensor gebildet und mit der Auswerteeinheit verbunden ist, wobei in dem Gehäusemantel eine Antriebs-Dehnungskraft vorliegt, die sich als Reaktionskraft aus einer Vorspannkraft einer einen Antriebskolben des Antriebs rückstellenden Feder generiert, und wobei die Hub-Messeinrichtung an dem Gehäusemantel angeordnet und dazu ausgelegt ist, die Antriebs-Dehnungskraft zu messen.

Über die bekannte Federkennlinie der Feder kann ein eindeutiger Zusammenhang zwischen der gemessenen Antriebs-Dehnungskraft, der dehnenden Kraft an sich, und dem Federweg hergestellt und somit die Hublage des Schließgliedes ermittelt werden. Die Hub-Messeinrichtung mit dem vorstehend vorgeschlagenen Merkmale begründet eine eigenständige Erfindung.

Da im Regelfall das Laternengehäuse in Form einer ersten Laternentraverse und einer dieser gegenüberliegenden zweiten Laternentraverse ausgebildet ist und sich die zu messende Reaktionskraft unter den gegebenen Herstellungs- und Betriebsbedingungen nicht gleichmäßig auf beide Laternentraversen verteilt, wird die Qualität der Messung der Reaktionskraft signifikant verbessert wenn in beiden Laternentraversen eine erfindungsgemäße Messeinrichtung angeordnet ist. Hierzu schlägt die Erfindung vor, dass das Laternengehäuse eine erste Laternentraverse und eine dieser gegenüberliegende zweite Laternentraverse aufweist, dass die erste Messeinrichtung an der ersten Laternentraverse angeordnet ist, dass eine zweite Messeinrichtung vorgesehen ist, die von wenigstens einem weiteren Dehnungssensor gebildet, an der zweiten Laternentraverse angeordnet und mit der Auswerteeinheit verbunden ist. Das Laternengehäuse kann prinzipiell auch mehr als zwei Laternentraverse aufweisen, die dann in gleicher Weise jeweils mit mindestens einem Dehnungssensor zu bestücken sind.

Weiterhin schlägt die Erfindung vor, dass in der ersten Messeinrichtung ein erster und ein zweiter Dehnungssensor, dass in der zweiten Messeinrichtung ein dritter und ein vierter Dehnungssensor, dass in der Hub-Messeinrichtung ein fünfter und ein sechster Dehnungssensor angeordnet sind, und dass von jedem Paar Dehnungssensoren der eine in Hubrichtung und der andere in orthogonaler Richtung hierzu angeordnet sind. Die orthogonale Anordnung der Dehnungssensoren ermöglicht auf sehr einfache und in an sich bekannter Weise eine Temperaturkompensation, wobei die Messsignale der Dehnungssensoren in ebenfalls an sich bekannter Weise in einer sogenannten Brückenschaltung unterschiedlicher Ausgestaltung ausgewertet werden. Die Auswerteeinheit kann in Gestalt einer internen Auswerteeinheit in der Steuereinheit des Hubventils oder auch in einer externen Auswerteeinheit außerhalb des Hubventils angeordnet sein.

Die Dehnungssensoren in der bevorzugten Ausgestaltung als DMS sind am Laternengehäuse kraftschlüssig angebracht. Aus der Längenänderung dieser Dehnungsmessstreifen kann auf die zeit- und damit hubabhängige Spannung im Laternenquerschnitt und damit auf die gleichfalls zeit- und hubabhängige Reaktionskraft und damit auf die

Stellkraft in der Verstellstange geschlossen werden, da der kraftübertragende Querschnitt und das Material des Laternengehäuses bekannt sind. In der Regel ist die Kenntnis des Betrages der Reaktionskraft oder der Stellkraft nicht erforderlich, da bei der erfindungsgemäßen vergleichenden Betrachtung der Messergebnisse die den Kräften linear proportionalen, als Spannungswerte ausgegebenen Dehnungswerte hinreichend sind. Bei der Hub-Messeinrichtung kann bei Verwendung von DMS und Kenntnis der Federkennlinie der jeweilige Hub ermittelt werden.

Das vorgeschlagene Diagnose-Verfahren für Hubventile und die Messeinrichtung zur Durchführung des Verfahrens in den jeweiligen Ausbildungsformen der Unteransprüche findet Anwendung auf ein Absperrventil mit einem einzigen Schließglied nach Anspruch 11 oder auf ein Doppelsitzventil nach Anspruch 12 oder auf ein sitzreinigungsfähiges Doppelsitzventil nach Anspruch 13 oder auf ein Doppeldichtventil nach Anspruch 14.

## KURZBESCHREIBUNG DER ZEICHNUNGEN

Während die Erfindung in den verschiedensten Verfahrensvarianten eines Diagnose-Verfahrens für Hubventile realisiert ist, werden in den Figuren der Zeichnung am Beispiel von zwei sich grundsätzlich in ihrer Schließgliedkonfiguration unterscheidenden Hubventilen, nämlich anhand eines Absperrventils mit einem einzigen Schließglied und eines Doppelsitzventils mit zwei unabhängig voneinander betätigbaren Schließgliedern, wobei beim Doppelsitzventil wiederum zwei unterschiedliche Schließglied- und Dichtungskonfigurationen dargestellt werden, die ermittelbaren Kraft-Zeit-Verläufe F2 = f(t) und Kraft-Hub-Verläufe F2 = f(h) einer Reaktionskraft F2 und ihre aufgabengemäße Deutung mit Blick auf den Zustand des jeweiligen Hubventils dargestellt. Des Weiteren werden zwei vorteilhafte Ausführungsformen einer Messeinrichtung dargestellt, die mittels Dehnungssensoren eine von der Stellkraft F1 der Ventilstange generierte Reaktionskraft F2 messen können. In Verbindung mit einer weiterhin dargestellten Hub-Messeinrichtung, die gleichfalls Dehnungssensoren beinhaltet und als indirekter Wegaufnehmer arbeitet, lässt sich dem Kraft-Zeit-Verlauf F2 = f(t) der Reaktionskraft F2 ein Hub-Zeit-Verlauf h = f(t) zuordnen. Die dargestellten und nachfolgend beschrieben Verfahrensvarianten und Ausführungsformen stellen nur Beispiele für die Erfindung dar, die Erfindung ist aber nicht auf diese speziell dargestellten Beispiele beschränkt.

Es zeigen

- **Figur 1** im unteren Teil im Meridianschnitt und im oberen Teil in der Ansicht ein als Absperrventil mit einem einzigen Schließglied ausgebildetes Hubventil, wobei erfindungsgemäß zwei Messeinrichtungen mit Dehnungssensoren zur Ermittlung einer von der Stellkraft F1 der Ventilstange im Laternengehäuse generierten Reaktionskraft F2 und eine Messeinrichtung mit Dehnungssensoren zur indirekten Ermittlung des Hubs am federschließenden Hubventil angeordnet sind und wobei aufgezeigt ist, welche einzelnen, an der Ventilstange angreifenden Kräfte (F3, F4, F5) diese Stellkraft F1 überwinden muss;
- **Figur 1a** einen Meridianschnitt durch einen Antrieb mit einer Messeinrichtung mit Dehnungssensoren zur indirekten Ermittlung des Hubs gemäß Figur 1, wobei die wirkenden Kräfte dargestellt sind;
- **Figur 1b** ein dem Antrieb gemäß Figur 1a zugeordnetes Federdiagramm, das den Zusammenhang zwischen der durch die Dehnungssensoren am Gehäusemantel des Antriebs gemessenen Antriebs-Dehnungskraft FA und dem aktuellen Hub h(FA) aufzeigt, wobei auf der Ordinate der Hub h und auf der Abszisse die Antriebs-Dehnungskraft FA aufgetragen sind;
- **Figur 2** im unteren Teil im Meridianschnitt und im oberen Teil in der Ansicht ein als Doppelsitzventil erster Art mit zwei unabhängig voneinander betätigbaren Schließgliedern (Sitzteller) ausgebildetes Hubventil, wobei erfindungsgemäß zwei Messeinrichtungen mit Dehnungssensoren zur Ermittlung einer von der Stellkraft F1 der Ventilstange im Laternengehäuse generierten Reaktionskraft F2 und eine Messeinrichtung mit Dehnungssensoren zur indirekten Ermittlung des Hubs am federschließenden Hubventil angeordnet sind und wobei aufgezeigt ist, welche einzelnen, an der Ventilstange angreifenden Kräfte (F3, F4, F5.1, F5.2) diese Stellkraft F1 überwinden muss;
- **Figur 3** in einem Diagramm den jeweiligen Kraft-Zeit-Verlauf F2 = f(t) der Reaktionskraft F2 in jeder der beiden Laternentraversen eines Absperrventils gemäß **Figur 1,** wobei auf der Ordinate die Reaktionskraft F2 und auf der Abszisse die Zeit t aufgetragen sind;
- **Figur 4** in einem Diagramm den jeweiligen Kraft-Zeit-Verlauf F2 = f(t) der Reaktionskraft F2 in jeder der beiden Laternentraversen eines Doppelsitzventils erster Art gemäß **Figur 2,** wobei auf der Ordinate die Reaktionskraft F2 und auf der Abszisse die Zeit t aufgetragen sind;
- **Figur 4a** das Diagramm gemäß **Figur 4** zusätzlich mit einem Kraft-Zeit-Verlauf F2 = f(t) der Reaktionskraft F2 beim Einfahren in die Schließstellung für ein Doppelsitzventil zweiter Art mit einem als Schieberkolben ausgebildeten ersten Schließglied und einem als Sitzteller ausgebildeten zweiten Schließglied;
- **Figur 5** in einem Diagramm den indirekt ermittelten Hub-Zeit-Verlauf h(FA) = f(t) für einen Antrieb gemäß den **Figuren 1a, 1b** im Vergleich mit einem durch direkte Wegmessung ermittelten Hub-Zeit-Verlauf h = f(t);
- **Figur 6** in einem Diagramm den Kraft-Hub-Verlauf F2 = f(h) der Reaktionskraft F2 in einer Laternentraversen eines

Absperrventils gemäß **Figur 1** mit einer neuwertigen und intakten Sitzdichtung, wobei auf der Ordinate die Reaktionskraft F2 und auf der Abszisse der Hub h aufgetragen sind;

**Figur 6a** in einem Diagramm den Kraft-Hub-Verlauf F2 = f(h) der Reaktionskraft F2 in einer Laternentraversen eines Absperrventils gemäß **Figur 1** mit einer teilweise herausgerissenen Sitzdichtung;

**Figur 6b** in einem Diagramm den Kraft-Hub-Verlauf F2 = f(h) der Reaktionskraft F2 in einer Laternentraversen eines Absperrventils gemäß **Figur 1** mit einer vollständig entfernten Sitzdichtung und

**Figur 7** in einem Diagramm den Kraft-Hub-Verlauf F2 = f(h) der Reaktionskraft F2 in einer Laternentraversen eines Doppelsitzventils erster Art gemäß **Figur 2** mit einer neuwertigen und intakten Sitzdichtung, wobei auf der Ordinate die Reaktionskraft F2 und auf der Abszisse der Hub h aufgetragen sind.

## DETAILLIERTE BESCHREIBUNG

Im Vorstehenden wurde bereits hinreichend dargelegt, dass über das in den Figuren 1 und 2 dargestellte Kräftespiel im Laternengehäuse 4 Aussagen über den Betriebszustand des Hubventils 100 und seinen Zustand im Allgemeinen (Zustand beispielsweise der Sitzdichtung, Zustand der Stangendurchführung, Material der Sitzdichtung, u.a.) gemacht werden können. Die Beschreibung der Figuren 1 und 2 wird nachfolgend durch einen ergänzenden Abriss des jeweiligen Aufbaus des dargestellten Absperrventils 110 **(Figur 1)** und eines nicht dargestellten Doppeldichtventils 140 sowie des Doppelsitzventils 120, 130 **(Figur 2),** jeweils in Verbindung mit den erfindungsgemäßen Messeinrichtungen, vervollständigt.

### Absperrventil (Figur 1)

Das als Absperrventil 110 ausgebildete Hubventil 100 besteht im Wesentlichen aus dem Ventilgehäuse 1 mit dem ersten und dem zweiten Ventilgehäuseteil 1 a, 1b, dem translatorisch bewegbaren Schließglied 8*, das als Sitzteller, wie dargestellt, oder als Schieberkolben ausgebildet sein kann und in der Schließstellung SS des Absperrventils 110 das Überströmen von Fluiden von dem einen Ventilgehäuseteil 1a, 1b in das andere 1b, 1a durch eine diese Ventilgehäuseteile 1a, 1b miteinander verbindende Verbindungsöffnung verhindert (Anmerkung: nicht alle erwähnten Bauteile sind in den Figuren 1, 2 bezeichnet). Das Schließglied 8* wirkt mit seiner Sitzdichtung 16* mit der im zweiten Ventilgehäuseteil 1b unmittelbar oder mittelbar ausgebildeten Sitzfläche 12* zusammen, wobei im Ausführungsbeispiel die Sitzfläche 12* an einem Sitzring angeordnet ist, der radial innenseits die Verbindungsöffnung ausbildet. Das erste Ventilgehäuseteil 1 a ist an seiner dem Sitzring gegenüberliegenden Seite mit einem Gehäusedeckel mittels eines sogenannten Spannrings verschlossen.

An dem Schließglied 8* ist die Verstellstange 8a* befestigt, die aus dem zweiten Ventilgehäuseteil 1 b über eine dort angeordnete Stangendichtung dichtend herausgeführt ist. Sie durchdringt anschließend das Laternengehäuse 4, das an seinem dem zweiten Ventilgehäuseteil 1 b abgewandten Ende mit dem Antrieb 2 fest, vorzugsweise form- und kraftschlüssig, beispielsweise mittels eines Spannrings, verbunden ist und endet im Bereich des antriebsseitigen Endes des Laternengehäuses 4. Die Verstellstange 8a* ist im axialen Erstreckungsbereich des Laternengehäuses 4 mit der Kolbenstange 2.6 fest verbunden **(Figur 1a),** wobei letztere eine zweite Gehäusestirnfläche 2.3 des Antriebs 2 abgedichtet durchdringt und an ihrem anderen Ende in fester Verbindung an einem Antriebskolben 2.4 angreift. Die feste Verbindung zwischen dem Laternengehäuse 4 und dem zweiten Ventilgehäuseteil 1 b stellt beispielsweise ein weiterer Spannring her. Das Laternengehäuse 4 besteht aus den zwei einander gegenüberliegenden Laternentraversen 4a und 4b.

Der Antrieb 2 wird von einem Gehäusemantel 2.1 radial außenseits und laternengehäuseseitig von der zweiten Gehäusestirnfläche 2.3 berandet und an seiner anderen Stirnseite von einer ersten Gehäusestirnfläche 2.2 begrenzt. Der Antriebskolben 2.4 ist axial verschieblich und radial abgedichtet im Gehäusemantel 2.1 geführt und er bildet zwischen sich und der zweiten Gehäusestirnfläche 2.3 einen mit Druckmittel D, vorzugsweise Druckluft, beaufschlagbaren Druckmittelraum aus. Zwischen dem Antriebskolben 2.4 und der ersten Gehäusestirnfläche 2.2 ist die Feder 2.5 mit der Vorspannkraft FV angeordnet. Unter einer vom Druckmittel D auf den Antriebskolben 2.4 ausgeübten Druckmittelkraft F6 verschiebt sich dieser um den Hub h **(Figur 1b),** ausgehend von der sich auch im Gehäusemantel 2.1 abbildenden minimalen Vorspannkraft FVo, bis er nach Vollzug eines vollen Öffnungshubes H am Gehäusemantel 2.1 zur Anlage kommt. Während der Hubbewegung h zwischen der Schließstellung SS mit h= 0 und der vollen Offenstellung OS mit h = H wird in der Feder 2.5 zusätzlich zur minimalen Vorspannkraft FVo eine zusätzliche Rückstellkraft generiert, die sich als eine Antriebs-Dehnungskraft FA am Gehäusemantel 2.1 in Form einer Zugkraft abbildet. Diese Antriebs-Dehnungskraft FA und damit ihr zeitlicher Verlauf FA(t) werden über eine am Gehäusemantel 2.1 vorzugsweise kraftschlüssig angeordnete Hub-Messeinrichtung 3.3 gemessen. In der Hub-Messeinrichtung 3.3 befinden sich ein fünfter und einer sechster Dehnungssensor DS5, DS6, die dort orthogonal zueinander angeordnet sind. Aus der Federkennlinie der Feder 2.5 **(Figur 1b)** ergibt sich ein eindeutiger Zusammenhang zwischen der gemessenen Antriebs-Dehnungskraft FA und dem zugehörigen Hub h(FA) und somit der Hublage des Schließgliedes 8*.

Auf der ersten Gehäusestirnfläche 2.2 ist eine Steuereinheit 2a angeordnet, der das Druckmittel D zur Beaufschlagung des Antriebs 2 zugeführt werden kann. Die Zufuhr des Druckmittels D kann aber auch direkt in den Antrieb 2 erfolgen. Eine Auswerteeinheit 2b für am Absperrventil 110 angeordnete Dehnungssensoren DS1 bis DS6 kann als interne Auswerteeinheit 2b.1 in der Steuereinheit 2a oder als externe Auswerteeinheit 2b.2 in der Umgebung des Absperrventils 110 angeordnet sein.

Die Öffnungsbewegung des Absperrventils 110 wird aus der dargestellten Schließstellung SS eingeleitet **(Figuren 1, 1a)** und die volle Offenstellung OS wird nach Vollzug des vollen Öffnungshubes H erreicht, wenn dem Druckmittelraum im Antrieb 2 das Druckmittel D über einen nicht dargestellten und bezeichneten Weg zugeführt wird. Im Zuge der Öffnungs- und Schließbewegung des Schließglieds 8* wird über den Antrieb 2 in der Verstellstange 8a* die als Aktionskraft fungierende Stellkraft F1 erzeugt, die die vorstehend genannten und erläuterten Kräfte, die Reibungskraft F3, die Strömungs- und/oder Druckkräfte F4 sowie die Reaktionskraft der Sitzdichtung F5, überwinden muss. Diese Stellkraft F1 bildet sich jeweils im Laternengehäuse 4 als Reaktionskraft F2 ab, wobei sich in der Ausführungsform des Latemengehäuses 4 mit zwei Laternentraversen 4a, 4b gemäß **Figur 1** diese Reaktionskraft F2 in einen ersten Reaktionskraftanteil F2a in der ersten Laternentraverse 4a und in einen zweiten Reaktionskraftanteil F2b in der zweiten Laternentraverse 4b aufteilt. Grundsätzlich ist dem Laternengehäuse 4 eine Messeinrichtung 3 mit zwei Dehnungssensoren DS1, DS2 zugeordnet, deren Messsignale in der Auswerteeinheit 2b verarbeitet werden. Im Ausführungsbeispiel nach **Figur 1** ist eine erste Messeinrichtung 3.1 vorzugsweise kraftschlüssig an der ersten Laternentraverse 4a angeordnet und die erste Messeinrichtung 3.1 weist den ersten und den zweiten Dehnungssensor DS1, DS2 auf, die mit der Auswerteeinheit 2b, und zwar in Form der internen Auswerteeinheit 2b.1 oder der externen Auswerteeinheit 2b.2, verbunden sind. Im Ausführungsbeispiel ist weiterhin eine zweite Messeinrichtung 3.2 an der zweiten Laternentraverse 4b angeordnet, wobei die zweite Messeinrichtung 3.2 einen dritten und einen vierten Dehnungssensor DS3, DS4 aufnimmt, die mit der Auswerteeinheit 2b, und zwar in Form der internen Auswerteeinheit 2b.1 oder der externen Auswerteeinheit 2b.2, verbunden sind. Über die fortlaufend gemessenen Reaktionskräfte F2 ergibt sich ein zeit- und hubabhängiger Verlauf dieser Reaktionskraft F2(t), F2(h), F2a(t), F2a(h), F2b(t) und F2b(h) und damit auch ein entsprechender Verlauf der Stellkraft F1(t) und F1(h).

<u>**Doppeldichtventil 140**</u>

Beim Doppeldichtventil 140 besitzt das einzige Schließglied 8* eine erste Sitzdichtung 16.1* und, axial beabstandet von letzterem, eine zweite Sitzdichtung 16.2*. Beiden Sitzdichtungen 16.1*, 16.2* kann eine gemeinsame Sitzfläche 12*, beispielsweise eine zylindrische, zugeordnet sein, in der sie beide radial abdichten. Es kann sich aber auch um unterschiedliche Sitzflächen handeln, die radial, axial oder kegelförmig orientiert sind. Hinsichtlich des Kräftespiels bleibt festzuhalten, dass die in **Figur 1** für das Absperrventil 110 dargestellten Kräfteverhältnisse, bis auf die Dichtungskraft F5, uneingeschränkt auf das Doppeldichtventil 140 übertragbar sind. An die Stelle der Dichtungskraft F5 tritt nunmehr aus jeder der beiden Sitzdichtungen 16.1* und 16.2* eine zugeordnete Dichtungskraft, die, sofern sie zu unterschiedlichen Zeiten mit der gemeinsamen Sitzfläche 12* in Eingriff kommen, separat detektierbar sind. Aus Vorstehendem ergibt sich, dass das erfindungsgemäße Diagnose-Verfahren und die Messeinrichtung zu seiner Durchführung uneingeschränkt auch auf das Doppeldichtventil 140 anwendbar sind.

<u>**Doppelsitzventil 120, 130 (Figur 2)**</u>

Die Unterschiede zwischen dem Doppelsitzventil 120, 130 **(Figur 2)** gegenüber dem Absperrventil 110 **(Figur 1)** mit Blick auf die Schließglied- und die Sitzkonfiguration wurden vorstehend bereits aufgezeigt, ebenso die Kräfteverhältnisse zur Darstellung der Stellkraft F1 an der Kolbenstange 2.6 nach den Gleichungen (3) bis (5) (F1 = F1.1 + F1.2) und der daraus resultierenden Reaktionskraft F2 im Laternengehäuse 4 nach Gleichung (6) (F1 = F2).

Zur weiteren Nomenklatur ist anzumerken, dass auch die Reaktionskraft F2 mit Blick auf die beiden Schließglieder 6, 8 differenziert betrachtet werden kann. So setzt sich die Reaktionskraft F2 aus einer ersten Reaktionskraft F2.1, bedingt durch das erste Schließglied 6, und aus einer zweiten Reaktionskraft F2.2, bedingt durch das zweite Schließglied 8, zusammen, die durch die am Laternengehäuse 4 angeordnete Messeinrichtung 3 erfasst werden können. Entsprechende zeit- und hubabhängige Reaktionskraft-Verläufe sind mit F2.1(t), F2.1(h), F2.2(t) und F2.2(h) bezeichnet. In einer bevorzugten Ausführungsform besteht das Laternengehäuse 4 aus den vorbeschriebenen zwei Laternentraversen 4a, 4b, so dass durch die dort angeordnete erste und die zweite Messeinrichtung 3.1, 3.2 nunmehr an der ersten Laternentraverse 4a ein erster Reaktionskraftanteils F2.1a aus dem ersten Schließglied 6 und ein erster Reaktionskraftanteil F2.2a aus dem zweiten Schließglied 8 und an der zweiten Laternentraverse 4b ein zweiter Reaktionskraftanteil F2.1b aus dem ersten Schließglied 6 und ein zweiter Reaktionskraftanteil F2.2b aus dem zweiten Schließglied 8 gemessen werden kann. Entsprechende zeit- und hubabhängige Reaktionskraft-Verläufe sind mit F2.1a(t), F2.1a(h), F2.2a(t), F2.2a(h), F2.1b(t), F2.1b(h), F2.2b(t) und F2.2b(h) bezeichnet.

In der Steuereinheit 2a **(Figuren 1, 2)** für diese Hubventile 100, die bevorzugt auf der dem Hubventil 100

abgewandten Seite des Antriebs 2 an letzterem angeordnet ist, Kann sich ein prozessor befinden, an den die Dehnungssensoren DS1 bis DS6, beispielsweise in Form von Dehnungsmessstreifen (DMS), zur Überwachung des Ventilbetriebs angeschlossen sind. Die Steuereinheit 2a kann ferner eine Einrichtung zur Erfassung und Speicherung von Betriebsgrößen und Kennlinien des Hubventils 100 und eine Einrichtung zur Erfassung und Speicherung der digitalisierten Messsignale der Dehnungssensoren DS1 bis DS6 aufweisen. Sie kann aber auch zur direkten Hubmessung eine an sich bekannte Wegmesseinrichtung aufnehmen.

__Diagramme (Figuren 3 bis 7__

Im Diagramm der Figur 3 ist der mit der erfindungsgemäßen ersten Messeinrichtung 3.1 und der zweiten Messeinrichtung 3.2 gemessene zeitliche Verlauf F2(t) der Reaktionskraft F2 eines Absperrventils 110 mit einer neuwertigen Sitzdichtung 16* **(Figur 1)** für einen Schaltzyklus dargestellt, wobei der mit 4a gekennzeichnete Verlauf in der ersten Laternentraverse 4a und der mit 4b gekennzeichnete in der zweiten Laternentraverse 4b gemessen wurde. Der Schaltzyklus beginnt, bezogen auf den zeitlichen Ablauf und ausgehend von der Schließstellung SS mit h = 0 (linker Bereich der Messverläufe), mit einem Öffnungsvorgang, das Schließglied 8* verbleibt nach Vollzug der vollen Offenstellung H eine zeitlang in der Offenstellung OS (mittlerer Bereich der Messverläufe) und schließt dann wieder in der Schließstellung SS.

Ausgehend jeweils von einem positiven ersten Reaktionskraftanteil F2ao und einem positiven zweiten Reaktionskraftanteil F2bo in der Schließstellung SS und zu Beginn eines Betriebs- oder Lebensdauerzyklus, wobei es sich um Zugkräfte in den Laternentraversen 4a, 4b handelt, fällt der jeweilige Reaktionskraftanteil beim Entlasten der Sitzdichtung 16* und ihrem anschließenden Verlassen der zugeordneten Sitzfläche 12* **(Figur 1)** im Zuge der Öffnungsbewegung signifikant auf den Wert nahe null ab. Wenn sich die Sitzdichtung 16* von ihrer Sitzfläche 12* vollständig gelöst hat und sich das Schließglied 8* in die volle Offenstellung H bewegt, wirken nur noch negative Reaktionskräfte F2 (Druckkräfte), die im vorliegenden Falle aus der Reibungskraft F3 in der Durchführung der Verstellstange 8a* resultieren. Beim anschließenden Einfahren des Schließgliedes 8* aus der Offenstellung OS in die Schließstellung SS kehren sich die Kräfteverhältnisse sinngemäß um.

In der vollen Offenstellung OS mit h = H ist der Antriebskolben 2.4 im Antrieb 2 (Figur 1a) unter dem Druck des Druckmittels D, das die Druckmittelkraft F6 generiert, an einem Anschlag am Gehäusemantel 2.1 zur Anlage gelangt. Die Kolbenstange 2.6 und damit die Verstellstange 8a* sind frei von Kräften aus der Sitzdichtung 16* und der Stangendurchführung; es verbleiben allenfalls noch Strömungskräfte F4, so dass sich ein weitgehend kräftefreier Zustand in den Laternentraversen 4a, 4b erklärt (F2 = 0 für h = H). Am Anfang der Öffnungs- und am Ende der Schließphase (h $\rightarrow$ 0), wenn die Sitzdichtung 16* noch bzw. wieder gegen die Sitzfläche 12* verpresst wird, herrscht in der Verstellstange 8a* die größte Druckkraft F1 und damit in den Laternentraversen 4a, 4b die größte Zugkraft F2. In der Schließstellung SS des Hubventils (h = 0) wird das Schließglied 8*, wenn der Antriebskolben 2.4 nicht vom Druckmittel D beaufschlagt ist, über die Verstellstange 8a* mit der im Antrieb 2 vorgesehenen minimalen Vorspannkraft FVo der Feder 2.5 auf seine Sitzfläche 12* gedrückt. Die Stellkraft F1 und damit die Reaktionskraft F2 erreichen dann ihren maximalen Betrag (F1 = F2; F2 = F2o = F2ao + F2bo). Die voneinander abweichenden Kraftwerte in den an sich geometrisch identischen Laternentraversen 4a, 4b erklären sich aus Fertigungstoleranzen und herstellungs- und montagebedingten Eigenspannungen. Sofern die Sitzdichtung 16* unter dieser minimalen Vorspannkraft FVo in ihre Dichtungsnut ausweichen kann, legt sich das Schließglied 8* metallisch an die Sitzfläche 12* an. Im Zuge des Öffnungs- und des Schließhubes ist die Feder 2.5 über die minimale Vorspannkraft FVo hinaus mit einer Vorspannkraft FV = FVo + FA vorgespannt (s. Figuren 1a, 1b), wobei es sich bei FA um die Antriebs-Dehnungskraft handelt. Die Antriebs-Dehnungskraft FA tritt nicht als Teil der Reaktionskraft F2 in Erscheinung; sie findet innerhalb des Antriebs 2 ihre Gegenkräfte.

Der Kraft-Zeit-Verlauf F2 = f(t) zwischen dem Anfang des Öffnens und dem Ende des Schließens des Hubventils 110 ist signifikant und typisch für den jeweiligen Zustand des Hubventils, insbesondere im Bereich seiner Sitzdichtung 16* und der Durchführung der Verstellstange 8a* im Bereich des zweiten Ventilgehäuseteils 1 b.

Damit ein Vergleich der Kraft-Zeit-Verläufe F2(t) von Schaltzyklen, die zu unterschiedlichen Zeiten der Betriebs- oder Lebensdauer des Hubventils 100 gemessen wurden, auf einfache und eindeutige Weise möglich ist, ist es zweckmäßig, zur Sicherstellung einer Vergleichbarkeit die gemessenen Kraft-Zeit-Verläufe der Stellkraft F1(t) oder der Reaktionskraft F2(t) jeweils bezüglich ihrer Kräfte und der zugeordneten Zeiten zu normieren. Der Kraft-Zeit-Verlauf F2(t) nach Figur 3 könnte beispielsweise mit Blick auf die Kräfte F2 mittels des ersten Reaktionskraftanteils F2ao und F2bo in der Schließstellung und zu Beginn des Betriebs- oder Lebensdauerzyklus normiert werden. Die den jeweils normierten Kraftwerten zugeordneten Zeiten t ließen sich beispielsweise mittels einer im Zusammenhang mit Figur 5 nachstehend erläuterten, zugeordneten Öffnungszeit t1 oder einer Schließzeit t2 normieren.

Im Diagramm der Figur 4 ist der mit der erfindungsgemäßen ersten Messeinrichtung 3.1 und der zweiten Messeinrichtung 3.2 gemessene zeitliche Verlauf F2(t) der Reaktionskraft F2 eines Doppelsitzventils 120, 130 mit zwei neuwertigen Sitzdichtungen 14, 16 (Figur 2) für einen Schaltzyklus dargestellt, wobei wiederum der mit 4a gekennzeichnete Verlauf in der ersten Laternentraverse 4a und der mit 4b gekennzeichnete in der zweiten Laternentraverse 4b

gemessen wurde. Bezüglich weiterer Einzelheiten des Schaltzyklus wird auf die Beschreibung zu Figur 3 verwiesen. Im Verlauf des jeweiligen Öffnungs- und des Schließvorganges zeigt sich ein signifikanter Unterschied zum jeweiligen Verlauf beim Absperrventil 110. Man erkennt in Verbindung mit Figur 2, dass beim Öffnungshub in der ersten Laternentraverse 4a zuerst der erste Reaktionskraftanteil F2.1a, hervorgerufen durch das erste Schließglied 6, abgebaut wird, weil dessen erste Sitzdichtung 14, analog zur Sitzdichtung 8* des Absperrventils 110, beim Verlassen der zugeordneten ersten Sitzfläche 10 dekomprimiert wird. Alsdann wird bei der weiteren Öffnungsbewegung, wenn auch die zweite Sitzdichtung 16 ihre zugeordnete zweite Sitzfläche 12 verlässt, der erste Reaktionskraftanteil F2.2a, hervorgerufen durch das zweite Schließglied 8, abgebaut. Für die zweite Laternentraverse 4b ergeben sich sinngemäß vergleichbare Verhältnisse. Die mit den erfindungsgemäßen Messeinrichtungen 3.1 und 3.2 gemessenen Kraft-Zeit-Verläufe F2(t) der Reaktionskraft F2 erlauben somit eine selektive Detektierung und Diagnose der beiden Sitzdichtungen 14, 16 und der diese unmittelbar oder mittelbar aufnehmenden Bauteile.

Das Diagramm der Figur 4a zeigt, ergänzend zum Diagramm gemäß Figur 4, einen Kraft-Zeit-Verlauf F2(t) der Reaktionskraft F2 beim Einfahren in die Schließstellung SS für ein Doppelsitzventil zweiter Art (s. die in Figur 4 eingeblendete kleine Abbildung) mit einem als Schieberkolben ausgebildeten ersten Schließglied 6, gemessen beispielsweise an der ersten Laternentraverse 4a. Im Bereich a des Kurvenverlaufs setzt die erste Sitzdichtung 14 des Schließgliedes 6 auf den Rand der zylindrischen ersten Sitzfläche 10 auf. Nach dem Eintauchen in die erste Sitzfläche 10 verfährt die erste Sitzdichtung 14 gleitend in dieser und es kommt während dieser Verfahrbewegung zu einer Reduzierung der Reaktionskraft auf den ersten Reaktionskraftanteil F2.1a (Bereich b), bis die zweite Sitzdichtung 16 auf die zugeordnete zweite Sitzfläche 12 aufsetzt. Dieses Aufsetzen und die sich anschließende Kompression der ersten Sitzdichtung 16 äußern sich in einem Kraftanstieg (Bereich c) um den Betrag des ersten Reaktionskraftanteils F2.2a aus dem zweiten Schließglied 8, der sich dem ersten Reaktionskraftanteil F2.1 a überlagert (Bereich d: F2.1a + F2.2a). Anschließen löst sich das erste Schließglied 6 vom zweiten Schließglied 8, das auf die zugeordnete zweite Sitzflächen 12 mit dem ersten Reaktionskraftanteil F2.2a (Vorspannkraft F2.2a der zweiten Feder 2.7) gepresst wird und dort verharrt. Wenn das erste Schließglied 6 seine Endlage in der zylindrischen ersten Sitzfläche10 erreicht hat, entfällt der aus der Verschiebebewegung resultierende erste Reaktionskraftanteil F2.1a und es verbleibt in der Schließstellung SS dauerhaft der aus der Vorspannkraft der zweiten Feder 2.7 resultierende zweite Reaktionskraftanteil F2.2a (Bereich e).

Damit ist wiederum gezeigt, dass auch für dieses Doppelsitzventil zweiter Art die mit den erfindungsgemäßen Messeinrichtungen 3.1 und 3.2 gemessenen Kraft-Zeit-Verläufe F2(t) der Reaktionskraft F2 eine selektive Detektierung und Diagnose der beiden Sitzdichtungen 14, 16 und der diese unmittelbar oder mittelbar aufnehmenden Bauteile erlauben.

Da sich die Kraftverläufe der vorstehend beschriebenen Doppelsitzventile erster und zweiter Art signifikant und unverwechselbar voneinander unterscheiden, ermöglicht das erfindungsgemäße Diagnose-Verfahren über die Ermittlung der zugeordneten Kraftverläufe eine Feststellung, um welche Doppelsitzventil-Bauart es sich handelt (automatisch Typisierung). Dieses Typisierungs-Ergebnis kann wiederum erfindungsgemäß dazu benutzt werden, bestimmte notwendige Voreinstellungen am Hubventil am Beginn seiner Betriebs- oder Lebensdauer vorzunehmen (z.B. Voreinstellung von Toleranzbereichen, Wartungsintervallen).

Mit der am Antrieb 2 erfindungsgemäß angeordneten Hub-Messeinrichtung 3.3 (Figuren 1a, 1b) lässt sich der Hub h(FA) des Hubventils 100 (110, 120, 130, 140) in Abhängigkeit von der Zeit indirekt ermitteln (h(1=A) = f(t)), wie dies in Figur 5 gezeigt und vorstehend bereits erläuter wurde. Der Hubverlauf h = f(t), ermittelt zeitgleich zum Hubverlauf h(FA) = f(t) mit einer an sich bekannten Wegmesseinrichtung, die vorzugsweise in der Steuereinheit 2a des Hubventils 100 angeordnet ist (Figuren 1, 2), zeigt eine Überraschende Übereinstimmung der beiden Verläufe. Das Erreichen der Offenstellung OS (Hubstellung b) oder das Verlassen der Offenstellung OS (Hubstellung c) ist jeweils durch eine signifikante, sprunghafte Änderung des Hubverlaufs h(FA) = f(t) gekennzeichnet. Diese Änderung erklärt sich aus den metallischen Anschlagbedingungen zwischen Antriebskolben 2.4 und Gehäusemantel 2.1 im Antrieb 2. Da auch die Schließstellung SS eindeutig aus dem Hubverlauf h(FA) = f(t) detektierbar ist (Hubstellungen a und d), lassen sich mit der erfindungsgemäßen Hub-Messeinrichtung 3.3 auch die Schließzeit t1 und die Öffnungszeit t2 des Hubventils 100 für weitere Diagnose-Schritte automatisch ermitteln.

Das Ergebnis einer Verknüpfung des nach Figur 3 an der ersten Laternentraverse 4a gemessenen Kraft-Zeit-Verlaufs F2 = f(t) mit dem beispielsweise nach Figur 5 direkt gemessenen Hub-Zeit-Verlauf h = f(t), wobei die Verläufe an einem Absperrventil 110 mit einer neuwertigen, intakten Sitzdichtung 16* ermittelt wurden, zeigt Figur 6. Der in sich geschlossene Kraft-Hub-Verlauf F2 = f(h) wird, wie alle weiteren diesbezüglichen Verläufe gemäß den Figuren 6a, 6b und 7, im Gegenuhrzeigersinn durchlaufen.

Wird unter den gleichen Mess- und Auswertungsbedingungen dieses Absperrventil 110 nunmehr mit einer teilweise herausgerissenen Sitzdichtung 16* bestückt, so ergibt sich ein aus Figur 6a ersichtliche Kraft-Hub-Verlauf F2 = f(h). Ein Absperrventil 110, das gänzlich ohne Sitzdichtung 16* geschaltet wird, zeigt unter den gleichen Mess- und Auswertungsbedingungen den in Figur 6b dargestellten Kraft-Hub-Verlauf F2(h).

Für ein Doppelsitzventil 120, 130 gemäß Figur 2 mit neuwertigen, intakten Sitzdichtungen 14, 16 liefern die erfindungsgemäßen Messeinrichtungen 3.1, 3.2 und eine direkte Wegmessung des Hubs unter den vorstehenden Mess-

und Auswertungsbedingungen den in Figur 7 dargestellten Kraft-Hub-Verlauf F2(h). Das jeweilige Einfahren der Schließglieder 6, 8 mit ihren zugeordneten Sitzdichtungen 14, 16 in die Sitzflächen 10, 12 und auch das jeweilige Ausfahren aus diesen Sitzflächen bilden sich in überraschend deutlicher Weise in dem Kurvenverlauf ab. Damit lassen sich mit Blick auf die Ergebnisse gemäß den Figuren 6a und 6b bei teilweise oder gänzlich entfernter Sitzdichtung 14 oder 16 mit Hilfe des erfindungsgemäßen Diagnose-Verfahrens der Zustand der Sitzdichtungen 14, 16, die Reibungsverhältnisse an den Durchführungen der Verstellstangen 6a, 8a, der Vollzug der Schließstellung SS und der vollen Offenstellung OS sowie bei einem sitzreinigungsfähigen Doppelsitzventil 130 auch die im Zuge der Sitzreinigung spaltweit geöffneten Sitzflächen 12 und 14 und somit der Vollzug des hierzu jeweils erforderlichen Teilhubs detektieren und diagnostizieren.

Die Messverläufe eines intakten, neuwertigen und normal arbeitenden Ventils mit einer neuen Sitzdichtung (Normal-Betriebszustand des Hubventils 100) gemäß den Figuren 3 bis 6 und Figur **7** unterscheiden sich mehr oder weniger signifikant von dem Messverlauf eines Hubventils 100 mit beschädigter Sitzdichtung (Figuren 6a, 6b) und/oder beschädigter Stangendurchführung. Die Unterschiede sind signifikant und mit den erfindungsgemäß vorgeschlagenen Vergleichskriterien eindeutig und reproduzierbar zu detektieren. Es kann die weitergehende Feststellung getroffen werden, dass sich durch die Messverläufe insbesondere Verschleiß, bedingt durch Korrosion, Kavitation, Erosion oder mechanische Beschädigung, des gesamten Hubventils 100, insbesondere aber der Sitzdichtung(en) und/oder der miteinander im Wechselspiel stehenden Bauteile, erkennen lassen. Damit lässt sich z.B. auch eine Vorhersage des Wartungszeitpunktes treffen.

Neben der Diagnose des Zustands des Hubventils 100 sind aber auch Druckschläge und -stöße an dem Schließglied 8* oder den Schließglieder 6, 8 im Hubventil 100 detektier- und protokollierbar, wodurch auch der Prozessablauf einer Prozessanlage, in der das Hubventil 100 zum Einsatz kommt, überwacht werden kann.

Besonders zielführend ist es, bei der vergleichenden Betrachtung von Kraft-Hub-Verläufen F2(h) aus Schaltzyklen das Vergleichskriterium des vorstehend bereits erläuterten Flächenintegrals, das die Kompressions- W12 oder Dekompressionsarbeit W21 an der Sitzdichtung 8*, 6, 8 darstellt, heranzuziehen. Durch Bildung des Flächenintegrals unter dem Kraft-Hub-Verlauf F2(h) und Vorgabe einer Abweichung lässt sich eine Beschädigung des Hubventils 100 erkennen, da sich die vorstehend genannten Unterschiede in diesem Flächenintegralwert signifikant ausprägen.

BEZUGSZEICHENLISTE DER VERWENDETEN ABKÜRZUNGEN

**Absperr- (Doppeldicht-) und Doppelsitzventil**

100 Hubventil, allgemein
110 Absperrventil (ein Schließglied, eine Sitzdichtung)
120 Doppelsitzventil (zwei unabhängig betätigbare Schließglieder mit je einer Sitzdichtung)
130 sitzreinigungsfähiges Doppelsitzventil (gegenüber 120 zusätzlich eine jeweils getrennt voneinander ansteuerbare Teiloffenstellung für jedes Schließglied aufweisend)
140 Doppeldichtventil (ein Schließglied mit zwei beabstandeten Sitzdichtungen)

1 Ventilgehäuse
1a erster Ventilgehäuseteil
1b zweiter Ventilgehäuseteil

2 Antrieb (druckmittelbeaufschlagter Feder-Kolben-Antrieb)
2.1 Gehäusemantel
2.2 erste Gehäusestirnfläche
2.3 zweite Gehäusestirnfläche
2.4 Antriebskolben
2.5 Feder
2.6 Kolbenstange

2a Steuereinheit
2b Auswerteeinheit, allgemein
2b.1 interne Auswerteeinheit
2b.2 externe Auswerteeinheit

3 Messeinrichtung, allgemein (zur Ermittlung des Kraft-Zeit-Verlaufs und/oder des Hub-Zeitverlaufs)

3.1 erste Messeinrichtung
3.2 zweite Messeinrichtung
3.3 Hub-Messeinrichtung

4 Laternengehäuse
4a erste Laternentraverse
4b zweite Laternentraverse

D Druckmittel

DS Dehnungssensor, allgemein
DS1 erster Dehnungssensor (z.B. Dehnungsmessstreifen DMS)
DS2 zweiter Dehnungssensor (z.B. Dehnungsmessstreifen DMS)
DS3 dritter Dehnungssensor (z.B. Dehnungsmessstreifen DMS)
DS4 vierter Dehnungssensor (z.B. Dehnungsmessstreifen DMS)
DS5 fünfter Dehnungssensor (z.B. Dehnungsmessstreifen DMS)
DS6 sechster Dehnungssensor (z.B. Dehnungsmessstreifen DMS)

F1 Stellkraft (Aktionskraft)
F1(t) Stellkraft F1 in Abhängigkeit von der Zeit t (Kraft-Zeit-Verlauf der Stellkraft F1)
F1(h) Stellkraft F1 in Abhängigkeit vom Hub h (Kraft-Hub-Verlauf der Stellkraft F1)
F1o Stellkraft F1 in der Schließstellung und zu Beginn des Betriebs- oder Lebensdauerzyklus

F2 Reaktionskraft
F2(t) Reaktionskraft F2 in Abhängigkeit von der Zeit t (Kraft-Zeit-Verlauf der Reaktionskraft F2)
F2(h) Reaktionskraft F2 in Abhängigkeit vom Hub h (Kraft-Hub-Verlauf der Reaktionskraft F2)
F2o Reaktionskraft F2 in der Schließstellung und zu Beginn des Betriebs- oder Lebensdauerzyklus
F2a erster Reaktionskraftanteil

F2ao erster Reaktionskraftanteil in der Schließstellung und zu Beginn des Betriebs- oder Lebensdauerzyklus
F2a(t, h) erster Reaktionskraftanteil, abhängig von Zeit t oder Hub h
F2b zweiter Reaktionskraftanteil
F2bo zweiter Reaktionskraftanteil in der Schließstellung und zu Beginn des Betriebs- oder Lebensdauerzyklus
F2b(t, h) zweiter Reaktionskraftanteil, abhängig von Zeit t oder Hub h

F3 Reibungskraft (in Durchführung der Verstellstange 8a*)
F4 Strömungs- und/oder Druckkräfte (auf das Schließglied 8*)
F5 Dichtungskraft (Reaktionskraft der Sitzdichtung(en))
F6 Druckmittelkraft

FV Vorspannkraft der Feder (für $h > 0$)
FVo minimale Vorspannkraft der Feder (für $h = 0$)

FA Antriebs-Dehnungskraft (am Gehäusemantel 2.1) (Überschuss gegenüber FVo und für $0 < h \leq H$; $FA = FV - FVo$)
FA(t) Antriebs-Dehnungskraft in Abhängigkeit von der Zeit t

H Öffnungshub (volle Offenstellung)
OS Offenstellung ($h = H$)
SS Schließstellung ($h = 0$)

W12 Kompressionsarbeit im Hubintervall $\Delta h = h1 - h2$
W21 Dekompressionsarbeit im Hubintervall $\Delta h = h2 - h1$

a, b, c, d, e Hubstellungen

h Hub (beliebiger Hub zwischen h= 0 und h= H)
h(t) Hub h in Abhängigkeit von der Zeit t (Hub-Zeit-Verlauf)
h(FA) Hub h ermittelt aus FA

∆h Verformungsweg der Sitzdichtung, Hubintervall (∆h = h1 - h2)

t Zeit
∆t Zeitintervall

t1 Öffnungszeit
t2 Schließzeit

y Ordinate ((+)y-Richtung nach oben)

**Absperrventil 110, (Doppeldichtventil 140)**

8* Schließglied
8a* Verstellstange
12* Sitzfläche
16* Sitzdichtung
(16.1*) erste Sitzdichtung
(16.2*) zweite Sitzdichtung

**Doppelsitzventil 120,130**

2.7 zweite Feder
6 erstes Schließglied
6a erste Verstellstange

7 Leckagehohlraum

8 zweites Schließglied
8a zweite Verstellstange (Hohlstange)

10 erste Sitzfläche
12 zweite Sitzfläche
14 erste Sitzdichtung
16 zweite Sitzdichtung
18 Mitteldichtung

F1.1 erste Stellkraft (erstes Schließglied 6)
F1.2 zweite Stellkraft (zweites Schließglied 8)

F2.1 erste Reaktionskraft (erstes Schließglied 6)
F2.1(t, h) erste Reaktionskraft, abhängig von Zeit t oder Hub h
F2.1a erster Reaktionskraftanteil (erstes Schließglied 6)
F2.1b zweiter Reaktionskraftanteil (erstes Schließglied 6)
F2.1a(t, h) erster Reaktionskraftanteil, abhängig von t, h (erstes Schließglied 6)
F2.1b(t, h) zweiter Reaktionskraftanteil, abhängig von t, h (erstes Schließglied 6)

F2.2 zweite Reaktionskraft (zweites Schließglied 8)
F2.2(t, h) zweite Reaktionskraft, abhängig von Zeit t oder Hub h
F2.2a erster Reaktionskraftanteil (zweites Schließglied 8)
F2.2b zweiter Reaktionskraftanteil (zweites Schließglied 8)
F2.2a(t, h) erster Reaktionskraftanteil, abhängig von t, h (zweites Schließglied 8)
F2.2b(t, h) erster Reaktionskraftanteil, abhängig von t, h (zweites Schließglied 8)

F3.1, F3.2 Reibungskraft (in Durchführung der Verstellstangen 6a, 8a)

F4.1, F4.2 Strömungs- und/oder Druckkräfte (auf die Schließglieder 6, 8)
F5.1, F5.2 Reaktionskraft der Sitzdichtungen 14, 16

## Patentansprüche

1. Diagnose-Verfahren für Hubventile, mit dem eine von einem Antrieb (2) des Hubventils (100) generierte, eine Aktionskraft darstellende Stellkraft (F1) für wenigstens ein Schließglied (8*) des Hubventils (100) in Form eines Kraft-Zeit-Verlaufs ermittelt wird, die Ermittlung der Stellkraft (F1) entweder unmittelbar oder an einer aus der Stellkraft (F1) im Hubventil (100) resultierenden Reaktionskraft (F2) vorgenommen wird, wobei die Ermittlung der Stellkraft (F1) oder der Reaktionskraft (F2) durch eine Messung der von dieser jeweils bewirkten dehnenden Verformungen erfolgt und der gemessene, aktuelle Kraft-Zeit-Verlauf der Stellkraft (F1(t)) oder der Reaktionskraft (F2(t)) gespeichert und einer Auswertung unterzogen wird,
**dadurch gekennzeichnet,**

    • **dass** zeitlich parallel zum Kraft-Zeit-Verlauf der Stellkraft oder der Reaktionskraft (F1(t); F2(t)) ein Hub-Zeit-Verlauf (h(t)) des Hubs (h) des wenigstens einen Schließglieds (8*) gemessen wird, dass der Kraft-Zeit-Verlauf der Stellkraft oder der Reaktionskraft (F1(t); F2(t)) und der Hub-Zeit-Verlauf (h(t)) miteinander verknüpft werden und daraus ein Kraft-Hub-Verlauf der Stellkraft oder der Reaktionskraft (F1(h); F2(h)) ermittelt wird,
    • **dass** der über die Betriebs- oder Lebensdauer des Hubventils (100) jeweils ermittelte, aktuelle Kraft-Hub-Verlauf der Stellkraft (F1(h) oder der Reaktionskraft F2(h)) eines Schaltzyklus mit einem früheren, gespeicherten verglichen wird,
    • **dass** aus dem Vergleich Abweichungen ermittelt werden,
    • **dass** innerhalb eines vorgegebenen Toleranzbereichs für diese Abweichungen letztere akzeptiert werden,
    • und **dass** bei Überschreitung des vorgegebenen Toleranzbereichs durch diese Abweichungen eine Meldung und/oder ein Steuersignal generiert werden/wird,
    • **dass** der Hub (h) durch eine diesen innerhalb des Hubventils (100) indirekt abbildende physikalische Größe ermittelt wird, und
    • **dass** als physikalische Größe eine durch die Erzeugung des Hubs (h) im Antrieb (2) hervorgerufene Dehnung herangezogen wird.

2. Diagnose-Verfahren für Hubventile nach Anspruch 1,
**dadurch gekennzeichnet,**
**dass** ein Schaltzyklus in zeitlicher Abfolge wenigstens aus einer Schließstellung (SS), einer Öffnungsbewegung, einer Offen- (OS) oder Teiloffenstellung und/oder wenigstens einer Offen- (OS) oder Teiloffenstellung, einer Schließbewegung und einer Schließstellung (SS) besteht.

3. Diagnose-Verfahren für Hubventile nach Anspruch 1 oder 2,
**dadurch gekennzeichnet,**
**dass** ein zu Beginn der Betriebs- oder Lebensdauer des Hubventils (100) gemessener, akzeptierter Schaltzyklus jeweils zum Vergleich herangezogen wird.

4. Diagnose-Verfahren für Hubventile nach Anspruch 1 oder 2,
**dadurch gekennzeichnet,**
**dass** der gemessene, aktuelle Schaltzyklus jeweils mit dem zuletzt gemessenen und akzeptierten verglichen wird.

5. Diagnose-Verfahren für Hubventile nach Anspruch 1 oder 2,
**dadurch gekennzeichnet,**
**dass** der gemessene, aktuelle Schaltzyklus jeweils mit dem Mittelwert einer vorbestimmten Anzahl von zuletzt gemessenen und akzeptierten Schaltzyklen verglichen wird.

6. Diagnose-Verfahren für Hubventile nach einem der vorhergehenden Ansprüche,
**dadurch gekennzeichnet,**
**dass** zum Vergleich der Kraft-Zeit-Verläufe der Stellkraft oder der Reaktionskraft ((F1(t); F2(t)) oder der Kraft-Hub-Verläufe der Stellkraft oder der Reaktionskraft (F1(h); F2(h)) wenigstens ein Vergleichskriterium aus den nachfolgend genannten herangezogen wird:

    • die Steigung

- die Krümmung
- der Betrag

an jeweils vorbestimmten diskreten Vergleichsstellen,

- die Betragsänderung
- das Flächenintegral

in jeweils vorbestimmten diskreten Zeit- oder Hubintervallen (Δt; Δh).

7. Diagnose-Verfahren für Hubventile nach einem der vorhergehenden Ansprüche,
**dadurch gekennzeichnet,**
**dass** der Kraft-Hub-Verlauf der Stellkraft oder der Reaktionskraft (F1 (h), F2(h)) zu Beginn der Betriebs- oder Lebensdauer des Hubventils (100; 110, 120, 130, 140) zur Erkennung der Bauart des Hubventils (100) herangezogen und das derart typisierte Hubventil (100) anschließend einer Voreinstellung mit Einstell- und/oder Überwachungsdaten unterzogen wird.

8. Messeinrichtung zur Durchführung des Diagnose-Verfahrens für Hubventile nach einem der vorhergehenden Ansprüche,
wobei die Messeinrichtung (3) an einem Hubventil (100) angeordnet ist, wobei das Hubventil (100) in einem Ventilgehäuse (1) wenigstens ein Schließglied (8*) aufweist, wobei das Ventilgehäuse (1) über ein Laternengehäuse (4) mit einem Antrieb (2) fest verbunden ist, wobei der Antrieb (2) als druckmittelbeaufschlagter Feder-Kolben-Antrieb ausgeführt ist, wobei wenigstens eine vom Antrieb (2) betätigbare Verstellstange (8a*) für das wenigstens eine Schließglied (8*) vorgesehen ist und wobei am Hubventil (100) eine der Messeinrichtung (3) zugeordnete Auswerteeinrichtung (2b) angeordnet ist, **dadurch gekennzeichnet,**
**dass** die Messeinrichtung (3) wenigstens aus einer ersten Messeinrichtung (3.1) besteht, die von wenigstens einem Dehnungssensor (DS) gebildet, an dem Laternengehäuse (4) angeordnet und mit der Auswerteeinheit (2b) verbunden ist,
**dass** an oder in einem Gehäusemantel (2.1) des Antriebs (2) eine Hub-Messeinrichtung (3.3) angeordnet ist, die von wenigstens einem weiteren Dehnungssensor (DS) gebildet und mit der Auswerteeinheit (2b) verbunden ist,
**dass** in dem Gehäusemantel (2.1) eine Antriebs-Dehnungskraft (FA) vorliegt, die sich als Reaktionskraft aus einer Vorspannkraft (FV) einer einen Antriebskolben (2.4) des Antriebs (2) rückstellenden Feder (2.5) generiert,
und **dass** die Hub-Messeinrichtung (3.3) dazu ausgelegt ist, die AntriebsDehnungskraft (FA) zu messen.

9. Messeinrichtung nach Anspruch 8,
**dadurch gekennzeichnet,**
**dass** das Laternengehäuse (4) eine erste Laternentraverse (4a) und eine dieser gegenüberliegende zweite Laternentraverse (4b) aufweist, dass die erste Messeinrichtung (3.1) an der ersten Laternentraverse (4a) angeordnet ist, dass eine zweite Messeinrichtung (3.2) vorgesehen ist, die von wenigstens einem weiteren Dehnungssensor (DS) gebildet, an der zweiten Laternentraverse (4b) angeordnet und mit der Auswerteeinheit (2b) verbunden ist.

10. Messeinrichtung nach Anspruch 8 oder 9,
**dadurch gekennzeichnet,**
**dass** in der ersten Messeinrichtung (3.1) ein erster und ein zweiter Dehnungssensor (DS1, DS2), dass in der zweiten Messeinrichtung (3.2) ein dritter und ein vierter Dehnungssensor (DS3, DS4), dass in der Hub-Messeinrichtung (3.3) ein fünfter und ein sechster Dehnungssensor (DS5, DS6) angeordnet sind, und dass von jedem Paar Dehnungssensoren (DS1, DS2; DS3, DS4; DS5, DS6) der eine in Hubrichtung und der andere in orthogonaler Richtung hierzu angeordnet sind.

11. Anwendung des Diagnose-Verfahrens für Hubventile nach einem der Ansprüche 1 bis 7 und der Messeinrichtung zur Durchführung des Verfahrens nach einem der Ansprüche 8 bis 10 auf ein Absperrventil (110) mit einem einzigen Schließglied (8*).

12. Anwendung des Diagnose-Verfahrens für Hubventile nach einem der Ansprüche 1 bis 7 und der Messeinrichtung zur Durchführung des Verfahrens nach einem der Ansprüche 8 bis 10 auf ein Doppelsitzventil (120) mit zwei mittels des Antriebs (2) unabhängig voneinander betätigbaren Schließgliedern (6, 8), die zwischen sich einen Leckagehohlraum (7) einschließen, der über mindestens einen Verbindungsweg mit der Umgebung des Doppelsitzventils (120) verbunden ist.

**13.** Anwendung des Diagnose-Verfahrens für Hubventile nach einem der Ansprüche 1 bis 7 und der Messeinrichtung zur Durchführung des Verfahrens nach einem der Ansprüche 8 bis 10 auf ein sitzreinigungsfähiges Doppelsitzventil (130) mit zwei mittels des Antriebs (2) unabhängig voneinander betätigbaren Schließgliedern (6, 8), die zwischen sich einen Leckagehohlraum (7) einschließen, der über mindestens einen Verbindungsweg mit der Umgebung des Doppelsitzventils (120) verbunden ist, wobei die Schließglieder (6, 8) jeweils getrennt voneinander ansteuerbare Teiloffenstellungen aufweisen.

**14.** Anwendung des Diagnose-Verfahrens für Hubventile nach einem der Ansprüche 1 bis 7 und der Messeinrichtung zur Durchführung des Verfahrens nach einem der Ansprüche 8 bis 10 auf ein Doppeldichtventil (140) mit einem einzigen Schließglied (8*), das zwei axial voneinander beabstandete Sitzdichtungen (16.1*, 16.2*) aufweist, die zwischen sich und in Verbindung mit zugeordneten Sitzflächen und dem Schließglied (8*) einen Leckagehohlraum einschließen, der über mindestens einen Verbindungsweg mit der Umgebung des Doppeldichtventils (140) verbunden ist.

## Claims

**1.** A diagnostic method for poppet valves with which an actuating force (F1) representing an action force, generated by a drive (2) of the poppet valve (100), is determined for at least one closing element (8*) of the poppet valve (100) in the form of a force-time curve, the actuating force (F1) is either determined directly or from a reaction force (F2) resulting from the actuating force (F1) in the poppet valve (100), wherein the actuating force (F1) or the reaction force (F2) is determined by measuring the expanding deformations caused therefrom, and the measured, current force-time curve of the actuating force (F1(t)) or the reaction force (F2(t)) is saved and subjected to an evaluation, **characterized in that**

- a displacement-time curve (h(t)) of the displacement (h) of the at least one closing element (8*) is measured at the same time as the force-time curve of the actuating force or the reaction force (F1(t); F2(t)), that the force-time curve of the actuating force or the reaction force (F1(t); F2(t)) and the displacement-time curve (h(t)) are linked together, and a force-displacement curve of the actuating force or the reaction force (F1(h); F2(h)) is determined therefrom,
- that the current force-displacement curve of the actuating force (F1(h)) or of the reaction force (F2(h)) of a switching cycle respectively determined during the operation or service life of the poppet valve (100) is compared with a saved, earlier one,
- that deviations from the comparison are determined,
- that deviations within a predetermined tolerance range for these deviations are accepted,
- and that when these deviations exceed the predetermined tolerance range, a message and/or control signal is/are generated,
- the displacement (h) is determined by a physical quantity indirectly representing the displacement within the poppet valve (100), and
- an expansion caused by the generation of the displacement (h) in the drive (2) is used as a physical quantity.

**2.** The diagnostic method for poppet valves according to claim 1,
**characterized in that**
a switching cycle in a sequence over time consists of at least one closed position (SS), an opening movement, an open (OS) or partially open position, and/or at least one open (OS) or partially open position, a closing movement, and a closed position (SS).

**3.** The diagnostic method for poppet valves according to claim 1 or 2,
**characterized in that**
a switching cycle measured and accepted at the start of the operation or service life of the poppet valve (100) is always used for comparison.

**4.** The diagnostic method for poppet valves according to claim 1 or 2,
**characterized in that**
the measured current switching cycle is compared with the last measured and accepted one.

**5.** The diagnostic method for poppet valves according to claim 1 or 2,
**characterized in that**

the measured current switching cycle is compared with the average of a predetermined number of last measured and accepted switching cycles.

6. The diagnostic method for poppet valves according to one of the preceding claims,
**characterized in that**
at least one comparative criterion of those cited below is used for comparing the force-time curves of the actuating force or of the reaction force ((F1(t); F2(t)) or the force-displacement curves of the actuating force or of the reaction force ((F1(h); F2(h)):

• the slope
• the curvature
• the amount at predetermined, discrete comparative locations in each case,
• the change in the amount
• the surface integral at predetermined discrete time or displacement intervals ($\Delta t$; $\Delta h$) in each case.

7. The diagnostic method for poppet valves according to one of the preceding claims,
**characterized in that**
the force-displacement curve of the actuating force or the reaction force (F1(h), F2(h)) at the beginning of the operation or service life of the poppet valve (100; 110, 120, 130, 140) is used to identify the design of the poppet valve (100) and the poppet valve (100) typified in this manner is then subjected to a preliminary adjustment with setting and/or monitoring data.

8. A measuring device for carrying out the diagnostic method for poppet valves according to one of the preceding claims, wherein the a measuring device (3) is arranged on a poppet valve (100), wherein the poppet valve (100) in a valve housing (1) has at least one closing element (8*), wherein the valve housing (1) is securely connected to a drive (2) via a lantern housing (4), wherein the drive (2) is designed as a spring-piston drive supplied with pressurization medium, wherein at least one adjusting rod (8a*) actuatable by the drive (2) is provided for the at least one closing element (8*), and wherein an evaluation device (2b) assigned to the measuring device (3) is arranged on the poppet valve (100),
**characterized in that**
the measuring device (3) consists of at least one first measuring device (3.1) which is formed by at least one expansion sensor (DS), arranged on the lantern housing (4), and connected to an evaluation unit (2b),
that a displacement-measuring device (3.3), which is formed by at least one further expansion sensor (DS) and is connected to the evaluation unit (2b), is arranged on or in a housing casing (2.1) of the drive (2),
that in the housing casing (2.1) there is a drive-expansion force (FA) which is generated as a reaction force from a pretension force (FV) of a spring (2.5) resetting a drive piston (2.4) of the drive (2),
and that the displacement measuring device (3.3) is designed for the purpose to measure the drive expansion force (FA).

9. The measuring device according to claim 8,
**characterized in that**
the lantern housing (4) has a first lantern crossmember (4a) and a second lantern crossmember (4b) opposite it, that the first measuring device (3.1) is arranged on the first lantern crossmember (4a), and that a second measuring device (3.2) is provided that is formed by at least one additional expansion sensor (DS), is arranged on the second lantern crossmember (4b), and is connected to the evaluation unit (2b).

10. The measuring device according to claim 8 or 9,
**characterized in that**
a first and second expansion sensor (DS 1, DS2) are arranged in the first measuring device (3.1), that a third and fourth expansion sensor (DS3, DS4) are arranged in the second measuring device (3.2), that a fifth and sixth expansion sensor (DS5, DS6) are arranged in the displacement measuring device (3.3), and that one expansion sensor of each pair of expansion sensors (DS1, DS2; DS3, DS4; DS5, DS6) is arranged in the direction of displacement, and the other is arranged in an orthogonal direction thereto.

11. A use of the diagnostic method for poppet valves according to one of claims 1 to 7 and the measuring device for carrying out the method according to one of claims 8 to 10 with a shutoff valve (110) with a single closing element (8*).

**12.** The use of the diagnostic method for poppet valves according to one of claims 1 to 7 and the measuring device for carrying out the method according to one of claims 8 to 10 with a double seat valve (120) having two closing elements (6, 8) that are independently actuatable by means of the drive (2) and that enclose between themselves a leakage cavity (7) which is connected via at least one connecting path to the surrounding area of the double seat valve (120).

**13.** The use of the diagnostic method for poppet valves according to one of claims 1 to 7 and the measuring device for carrying out the method according to one of claims 8 to 10 with a double seat valve (130) capable of seat-cleaning having two closing elements (6, 8) that are independently actuatable by means of the drive (2) and that enclose between themselves a leakage cavity (7) which is connected via at least one connecting path to the surrounding area of the double seat valve (120), wherein the closing elements (6, 8) each have partially opened positions that can be controlled separately from each other.

**14.** The use of the diagnostic method for poppet valves according to one of claims 1 to 7, and the measuring device for carrying out the method according to one of claims 8 to 10 with a double seal valve (140) with a single closing element (8*), that has two seat seals (16.1*, 16.2*) that are axially distanced from each other, which enclose between themselves and in conjunction with assigned seat surfaces and the closing element (8*) a leakage cavity that is connected via at least one connecting path to the surrounding area of the double seal valve (140).

## Revendications

**1.** Procédé de diagnostic pour soupapes, avec lequel une force de réglage (F1), générée par un entraînement (2) de la soupape (100) et représentant une force d'action, pour au moins un organe de fermeture (8*) de la soupape (100) est déterminée sous la forme d'une courbe force-temps, la détermination de la force de réglage (F1) est entreprisse soit directement soit au niveau d'une force de réaction (F2) résultant de la force de réglage (F1) dans la soupape (100), la détermination de la force de réglage (F1) ou de la force de réaction (F2) s'effectuant par une mesure des déformations d'allongement respectivement provoquées par celle-ci, et la courbe force-temps actuelle mesurée de la force de réglage (F1(t)) ou de la force de réaction (F2(t)) étant enregistrée et soumise à une analyse,
**caractérisé en ce que**

- une courbe course-temps (h(t)) de la course (h) de l'organe de fermeture (8*) au moins au nombre de un est mesurée parallèlement dans le temps à la courbe force-temps de la force de réglage ou de la force de réaction (F1(t)) ; (F2(t)), la courbe force-temps de la force de réglage ou de la force de réaction (F1(t)) ; (F2(t)) et la courbe course-temps (h(t)) sont liées l'une à l'autre et qu'à partir de cela une courbe force-course de la force de réglage ou de la force de réaction (F1(h)) ; (F2(h)) est déterminée,
- **en ce que** la courbe force-course actuelle déterminée respectivement par le biais de la durée de fonctionnement ou de vie de la soupape (100), de la force de réglage (F1(h) ou de la force de réaction F2(h)) d'un cycle de commutation est comparée à une courbe force-course antérieure enregistrée,
- **en ce que** des divergences sont déterminées à partir de la comparaison,
- **en ce que** ces divergences sont acceptées à l'intérieur d'une plage de tolérance prédéfinie pour ces divergences,
- et **en ce qu'**en cas de dépassement de la plage de tolérance prédéfinie par ces divergences, un message et/ou un signal de commande est/sont généré(s),
- **en ce que** la course (h) est déterminée par une grandeur physique reproduisant indirectement cette course à l'intérieur de la soupape (100), et
- **en ce qu'**un allongement suscité par la production de la course (h) dans l'entraînement (2) est utilisé en tant que grandeur physique.

**2.** Procédé de diagnostic pour soupapes selon la revendication 1,
**caractérisé en ce**
**qu'**un cycle de commutation se compose, dans l'ordre chronologique, d'au moins une position de fermeture (SS), d'un mouvement d'ouverture, d'une position d'ouverture (OS) ou d'ouverture partielle et/ou d'au moins une position d'ouverture (OS) ou d'ouverture partielle, d'un mouvement de fermeture et d'une position de fermeture (SS).

**3.** Procédé de diagnostic pour soupapes selon la revendication 1 ou 2,
**caractérisé en ce**
**qu'**un cycle de commutation accepté mesuré au début de la durée de fonctionnement ou de vie de la soupape (100) est respectivement utilisé pour la comparaison.

4. Procédé de diagnostic pour soupapes selon la revendication 1 ou 2,
**caractérisé en ce que**
le cycle de commutation actuel mesuré est respectivement comparé au cycle de commutation mesuré en dernier et accepté.

5. Procédé de diagnostic pour soupapes selon la revendication 1 ou 2,
**caractérisé en ce que**
le cycle de commutation actuel mesuré est respectivement comparé à la valeur moyenne d'un nombre prédéfini de cycles de commutation mesurés en dernier et acceptés.

6. Procédé de diagnostic pour soupapes selon une des revendications précédentes, **caractérisé en ce que,**
pour la comparaison des courbes force-temps de la force de réglage ou de la force de réaction ((F1(t)) ; (F2(t)) ou des courbes force-course de la force de réglage ou de la force de réaction ((F1(h)) ; (F2(h)), il est utilisé au moins un critère de comparaison parmi ceux cités ci-dessous :

- la pente
- la courbure
- la valeur

à des emplacements de comparaison discrets prédéfinis,

- la variation de valeur
- l'intégrale de la surface

à des intervalles de temps ou de course ($\Delta t$ ; $\Delta h$) discrets respectivement prédéfinis.

7. Procédé de diagnostic pour soupapes selon une des revendications précédentes,
**caractérisé en ce que**
la courbe force-course de la force de réaction (F1(h)) ; (F2(h)) au début de la durée de fonctionnement ou de vie de la soupape (100 ; 110, 120, 130, 140) est utilisée pour l'identification du type de fabrication de la soupape (100), et **en ce que** la soupape (100) ainsi catégorisée est ensuite soumise à un préréglage avec des données de réglage et/ou de surveillance.

8. Dispositif de mesure pour la mise en oeuvre du procédé de diagnostic pour des soupapes selon une des revendications précédentes,
le dispositif de mesure (3) étant disposé sur une soupape (100), la soupape (100) présentant dans un boîtier des soupapes (1) au moins un organe de fermeture (8*), le boîtier des soupapes (1) étant raccordé de façon fixe à un entraînement (2) par le biais d'un boîtier à lanterne (4), l'entraînement (2) étant réalisé en tant qu'entraînement ressort-piston soumis à un moyen de pression, au moins une tige de réglage (8a*) pouvant être actionnée par l'entraînement (2) étant prévue pour l'organe de fermeture (8*) au moins au nombre de un, et un dispositif d'analyse (2b) affecté au dispositif de mesure (3) étant disposé sur la soupape (100),
**caractérisé en ce que**
le dispositif de mesure (3) se compose d'au moins un premier dispositif de mesure (3.1) qui est formé d'au moins un capteur d'allongement (DS), qui est disposé sur le boîtier à lanterne (4) et qui est raccordé à l'unité d'analyse (2b),
**en ce que**, sur ou dans une enveloppe de boîtier (2.1) de l'entraînement (2), il est disposé un dispositif de mesure de la course (3.3) qui est formé d'au moins un autre capteur d'allongement (DS) et est raccordé à l'unité d'analyse (2b),
**en ce que**, dans l'enveloppe de boîtier (2.1), il existe une force d'allongement d'entraînement (FA) qui est générée en tant que force de réaction à partir d'une force de prétension (FV) d'un ressort (2.5) rappelant un piston d'entraînement (2.4) de l'entraînement (2),
et **en ce que** le dispositif de mesure de la course (3.3) est conçu pour mesurer la force d'allongement d'entraînement (FA).

9. Dispositif de mesure selon la revendication 8,
**caractérisé en ce que**
le boîtier à lanterne (4) présente une première traverse de lanterne (4a) et une deuxième traverse de lanterne (4b) superposée à celle-ci, **en ce que** le premier dispositif de mesure (3.1) est disposé sur la première traverse de lanterne (4a), **en ce qu'**il est prévu un deuxième dispositif de mesure (3.2) qui est formé d'au moins un autre capteur d'allongement (DS), qui est disposé sur la deuxième traverse de lanterne (4b) et qui est raccordé à l'unité d'analyse

(2b).

**10.** Dispositif de mesure selon la revendication 8 ou 9,
**caractérisé en ce que,**
dans le premier dispositif de mesure (3.1), il est disposé un premier et un deuxième capteur d'allongement (DS1, DS2), **en ce que**, dans le deuxième dispositif de mesure (3.2), il est disposé un troisième et un quatrième capteur d'allongement (DS3, DS4), **en ce que**, dans le dispositif de mesure de la course (3.3), il est disposé un cinquième et un sixième capteur d'allongement (DS5, DS6), et **en ce que**, dans chaque paire de capteurs d'allongement (DS1, DS2 ; DS3, DS4 ; DS5, DS6), un capteur est disposé dans la direction de la course et l'autre capteur est disposé à angle droit par rapport à cette dernière.

**11.** Application du procédé de diagnostic pour soupapes selon une des revendications 1 à 7 et du dispositif de mesure pour la mise en oeuvre du procédé selon une des revendications 8 à 10 à une soupape d'arrêt (110) avec un unique organe de fermeture (8*).

**12.** Application du procédé de diagnostic pour soupapes selon une des revendications 1 à 7 et du dispositif de mesure pour la mise en oeuvre du procédé selon une des revendications 8 à 10 à une soupape à double siège (120) avec deux organes de fermeture (6, 8) pouvant être actionnés indépendamment l'un de l'autre au moyen de l'entraînement (2) et qui enferment entre eux une cavité de fuite (7) qui est raccordée à l'environnement de la soupape à double siège (120) par le biais d'au moins une voie de raccordement.

**13.** Application du procédé de diagnostic pour soupapes selon une des revendications 1 à 7 et du dispositif de mesure pour la mise en oeuvre du procédé selon une des revendications 8 à 10 à une soupape à double siège (130) avec capacité de nettoyage des sièges, avec deux organes de fermeture (6, 8) pouvant être actionnés indépendamment l'un de l'autre au moyen de l'entraînement (2) et qui enferment entre eux une cavité de fuite (7) qui est raccordée à l'environnement de la soupape à double siège (120) par le biais d'au moins une voie de raccordement, les organes de fermeture (6, 8) présentant respectivement des positions d'ouverture partielle pouvant être pilotées séparément les unes des autres.

**14.** Application du procédé de diagnostic pour soupapes selon une des revendications 1 à 7 et du dispositif de mesure pour la mise en oeuvre du procédé selon une des revendications 8 à 10 à une soupape à double étanchéité (140) avec un unique organe de fermeture (8*), qui présente deux joints d'étanchéité de siège (16.1*, 16.2*) espacés axialement l'un de l'autre qui enferment, entre eux et en liaison avec des surfaces de siège affectées et avec l'organe de fermeture (8*), une cavité de fuite qui est raccordée à l'environnement de la soupape à double étanchéité (140) par le biais d'au moins une voie de raccordement.

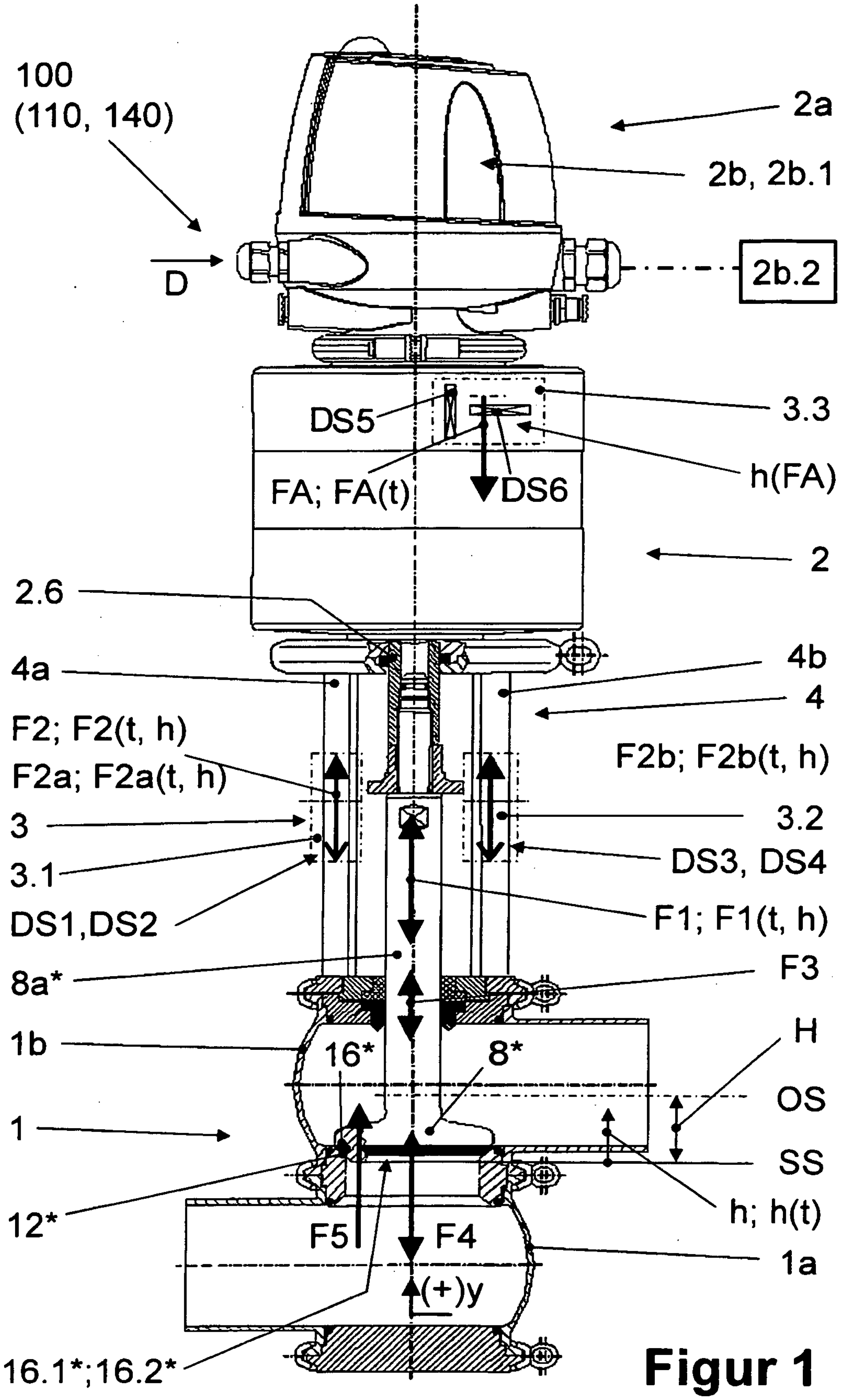
100
(110, 140)
2a
2b, 2b.1
D
2b.2
DS5
3.3
FA; FA(t)
DS6
h(FA)
2
2.6
4a
4b
4
F2; F2(t, h)
F2a; F2a(t, h)
F2b; F2b(t, h)
3
3.2
3.1
DS3, DS4
DS1,DS2
F1; F1(t, h)
8a*
F3
1b
16*
8*
H
OS
1
SS
12*
h; h(t)
F5
F4
1a
(+)y
16.1*;16.2*

Figur 1

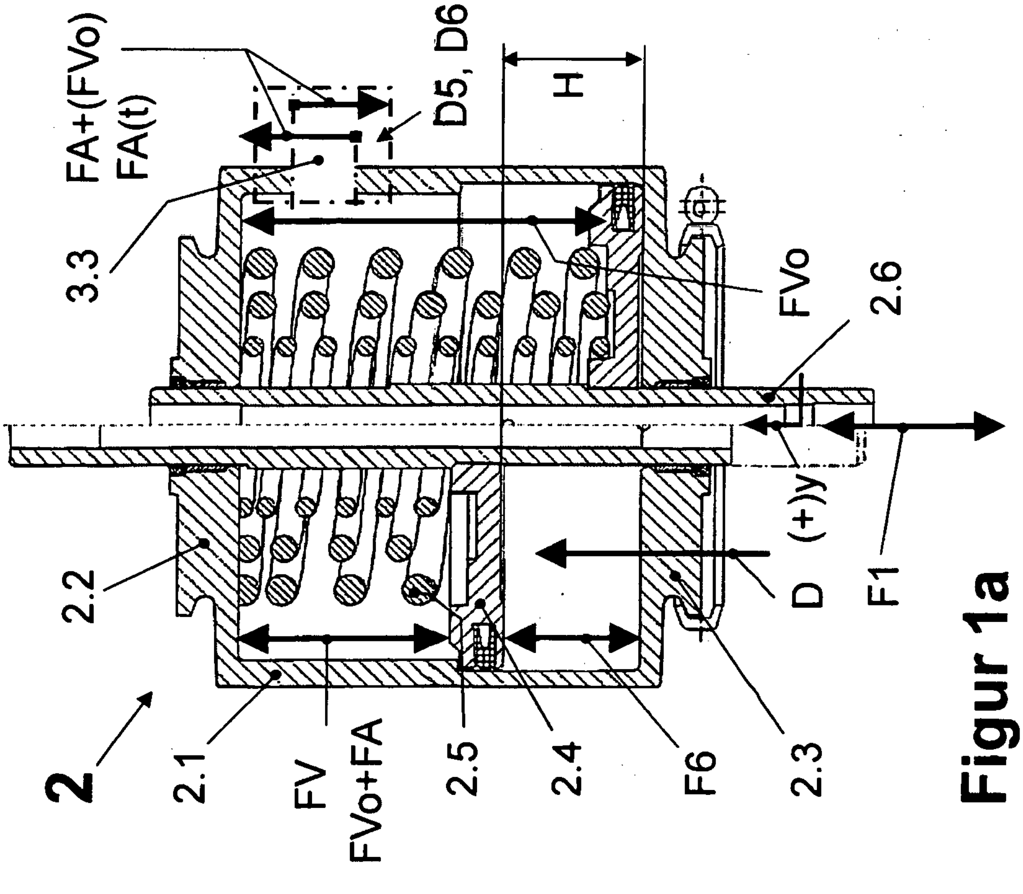


Figur 1a

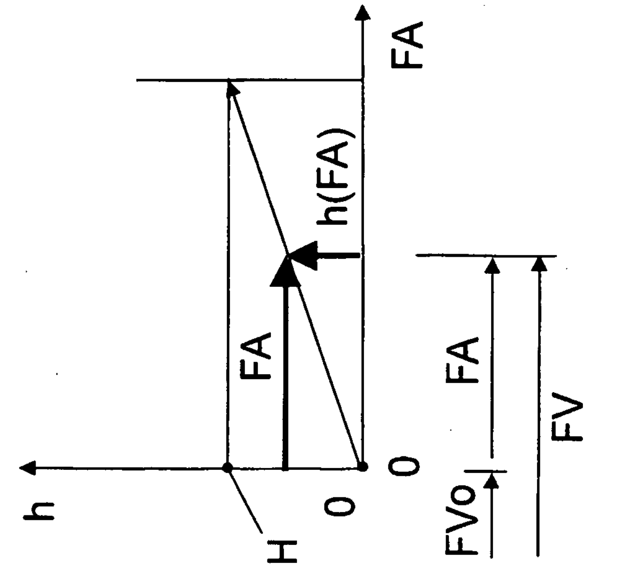


Figur 1b

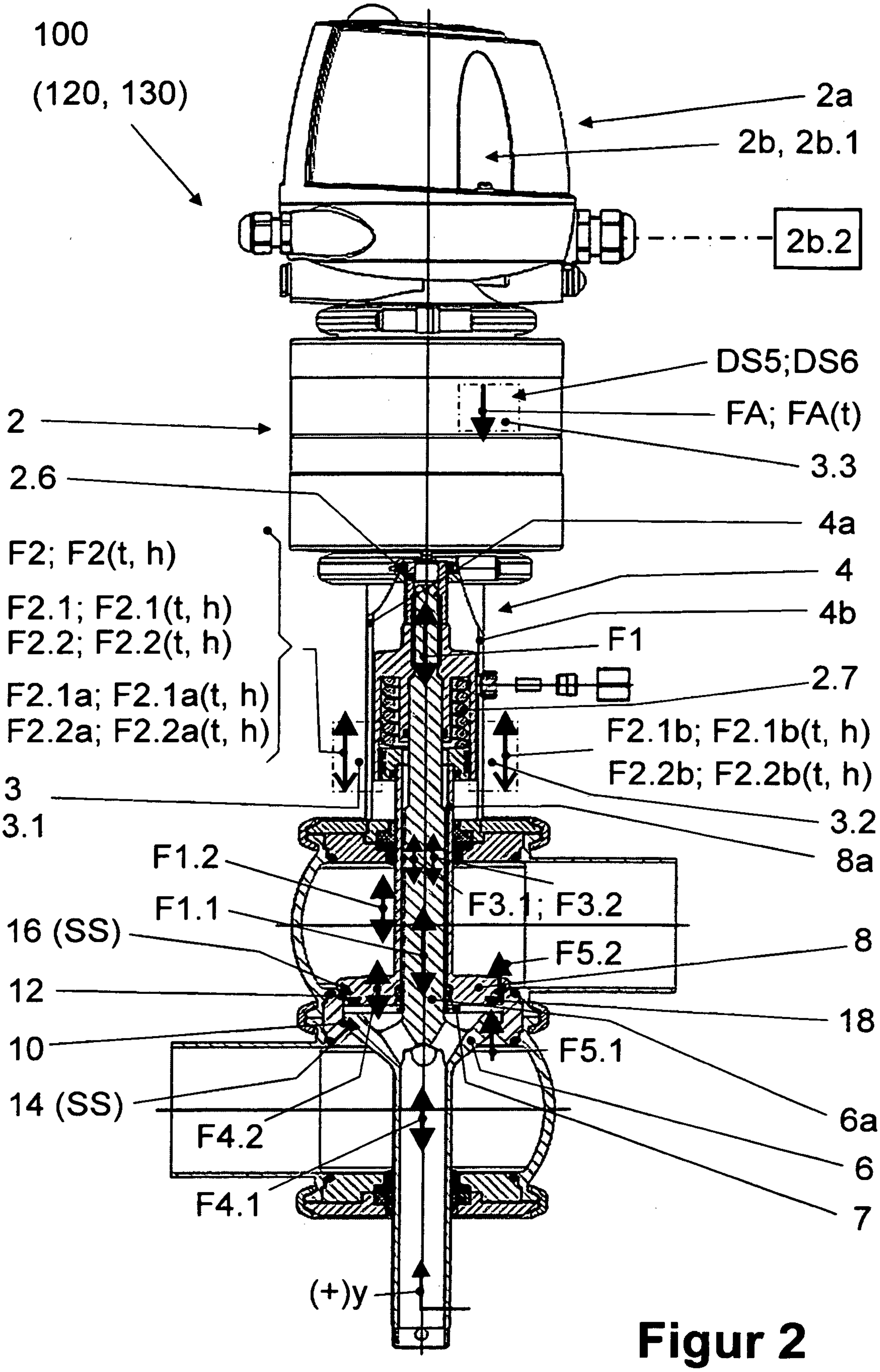
100
(120, 130)
2a
2b, 2b.1
2b.2
DS5;DS6
FA; FA(t)
2
3.3
2.6
4a
F2; F2(t, h)
4
F2.1; F2.1(t, h)
F2.2; F2.2(t, h)
4b
F1
2.7
F2.1a; F2.1a(t, h)
F2.2a; F2.2a(t, h)
F2.1b; F2.1b(t, h)
F2.2b; F2.2b(t, h)
3
3.1
3.2
8a
F1.2
F3.1; F3.2
F1.1
16 (SS)
F5.2
8
12
18
10
F5.1
14 (SS)
6a
F4.2
6
F4.1
7
(+)y

Figur 2

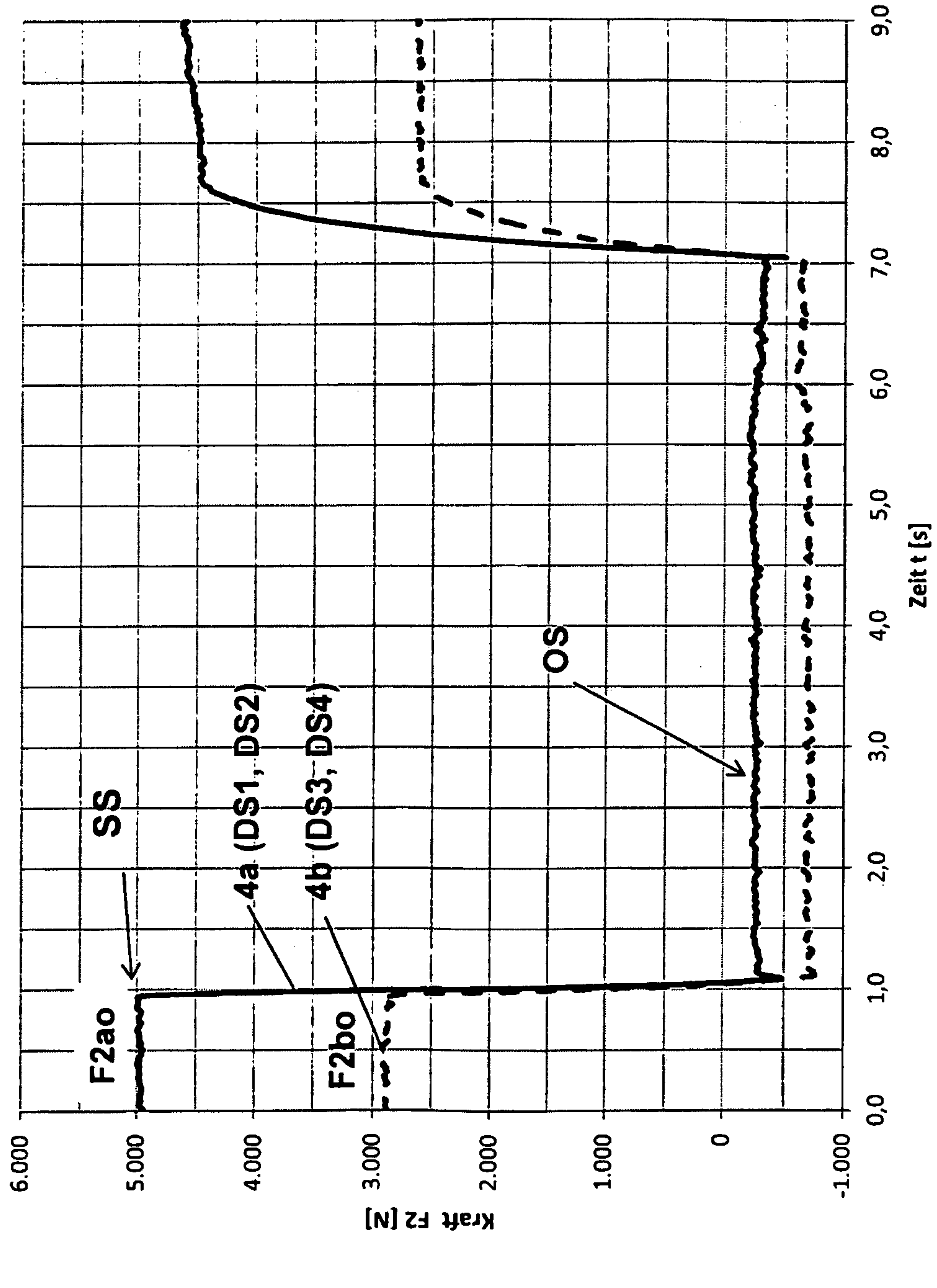
F2ao
SS
4a (DS1, DS2)
4b (DS3, DS4)
F2bo
OS
Kraft F2 [N]
6.000
5.000
4.000
3.000
2.000
1.000
0
-1.000
Zeit t [s]
0,0
1,0
2,0
3,0
4,0
5,0
6,0
7,0
8,0
9,0

Figur 3

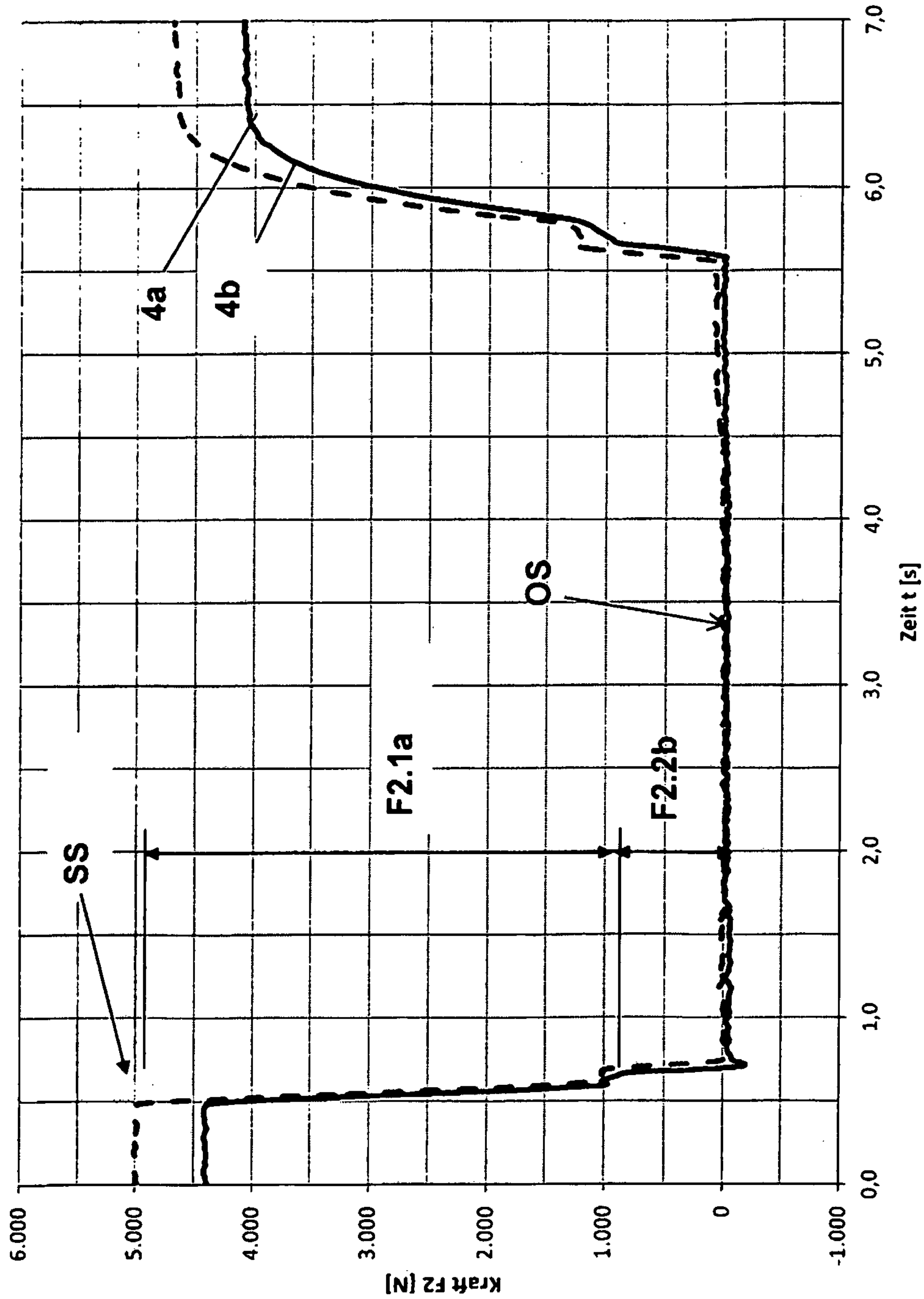
4a
4b
SS
F2.1a
F2.2b
OS
6.000
5.000
4.000
3.000
2.000
1.000
0
-1.000
Kraft F2 [N]
0,0
1,0
2,0
3,0
4,0
5,0
6,0
7,0
Zeit t [s]

Figur 4

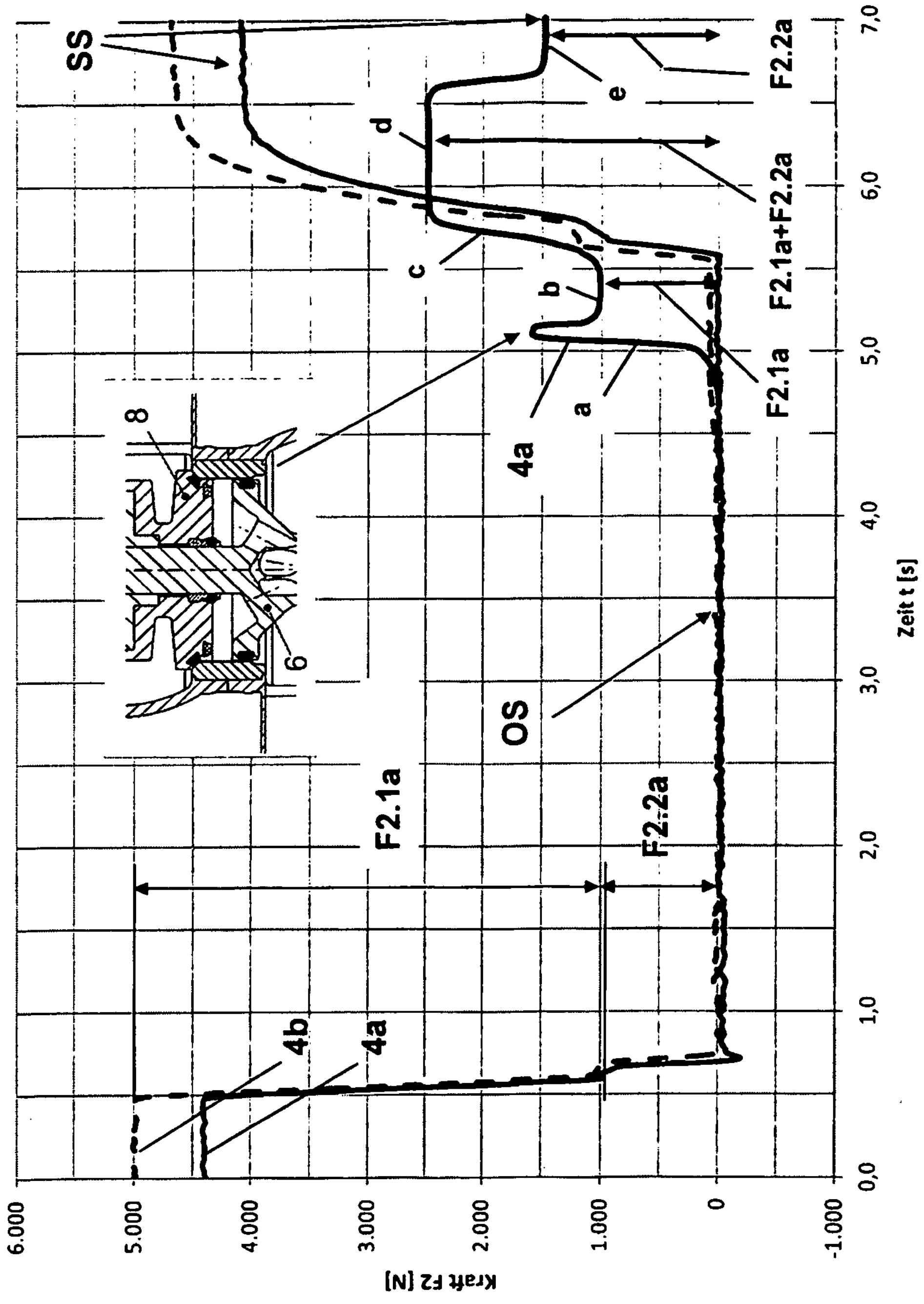
SS
d
e
c
b
F2.2a
F2.1a+F2.2a
F2.1a
a
4a
8
6
OS
F2.1a
F2.2a
4b
4a
6.000
5.000
4.000
3.000
2.000
1.000
0
-1.000
Kraft F2 [N]
0,0
1,0
2,0
3,0
4,0
5,0
6,0
7,0
Zeit t [s]

Figur 4a

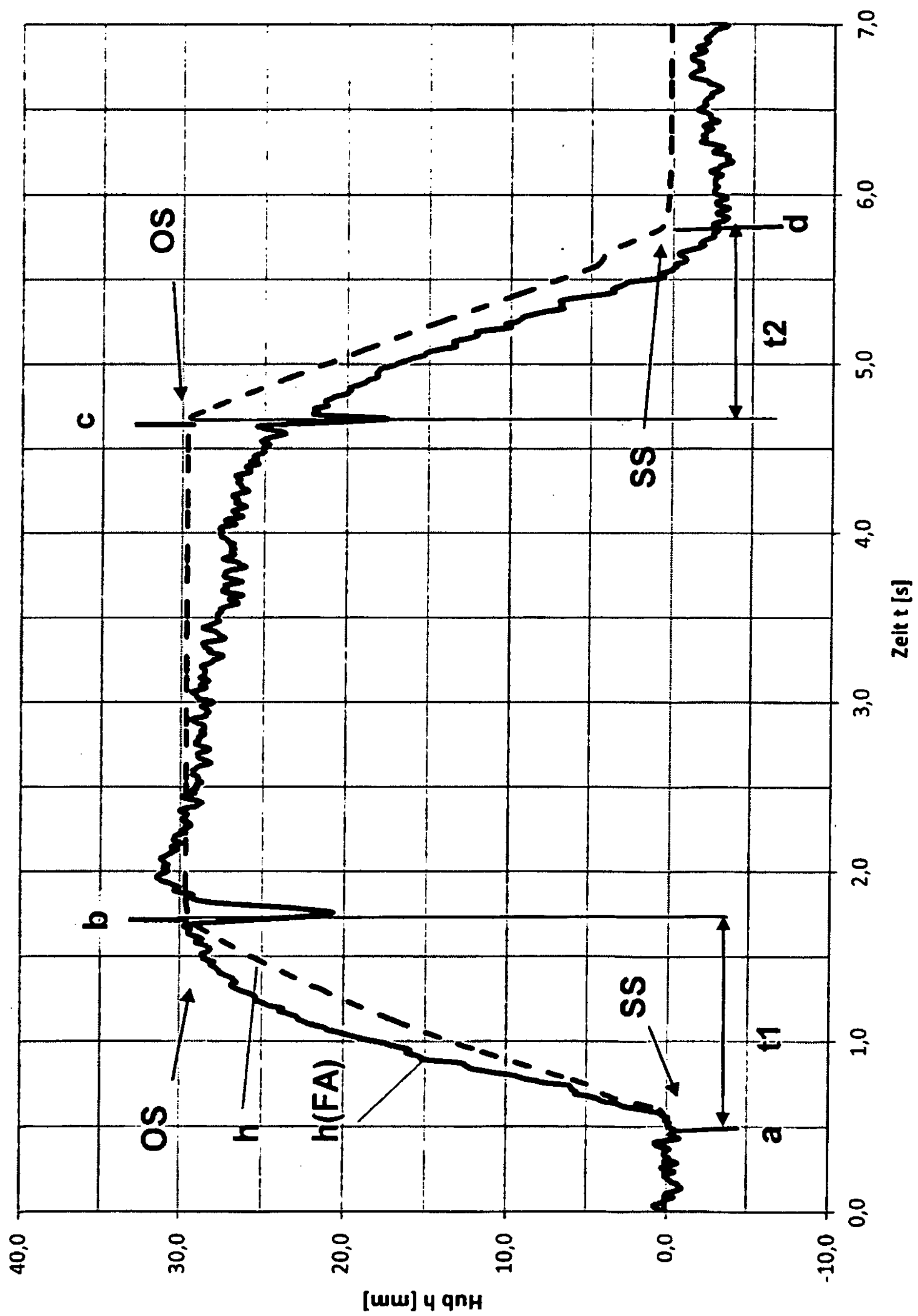
Hub h [mm]
40,0
30,0
20,0
10,0
0,0
-10,0
Zeit t [s]
0,0
1,0
2,0
3,0
4,0
5,0
6,0
7,0
OS
h
h(FA)
SS
a
b
t1
OS
c
SS
t2
d

Figur 5

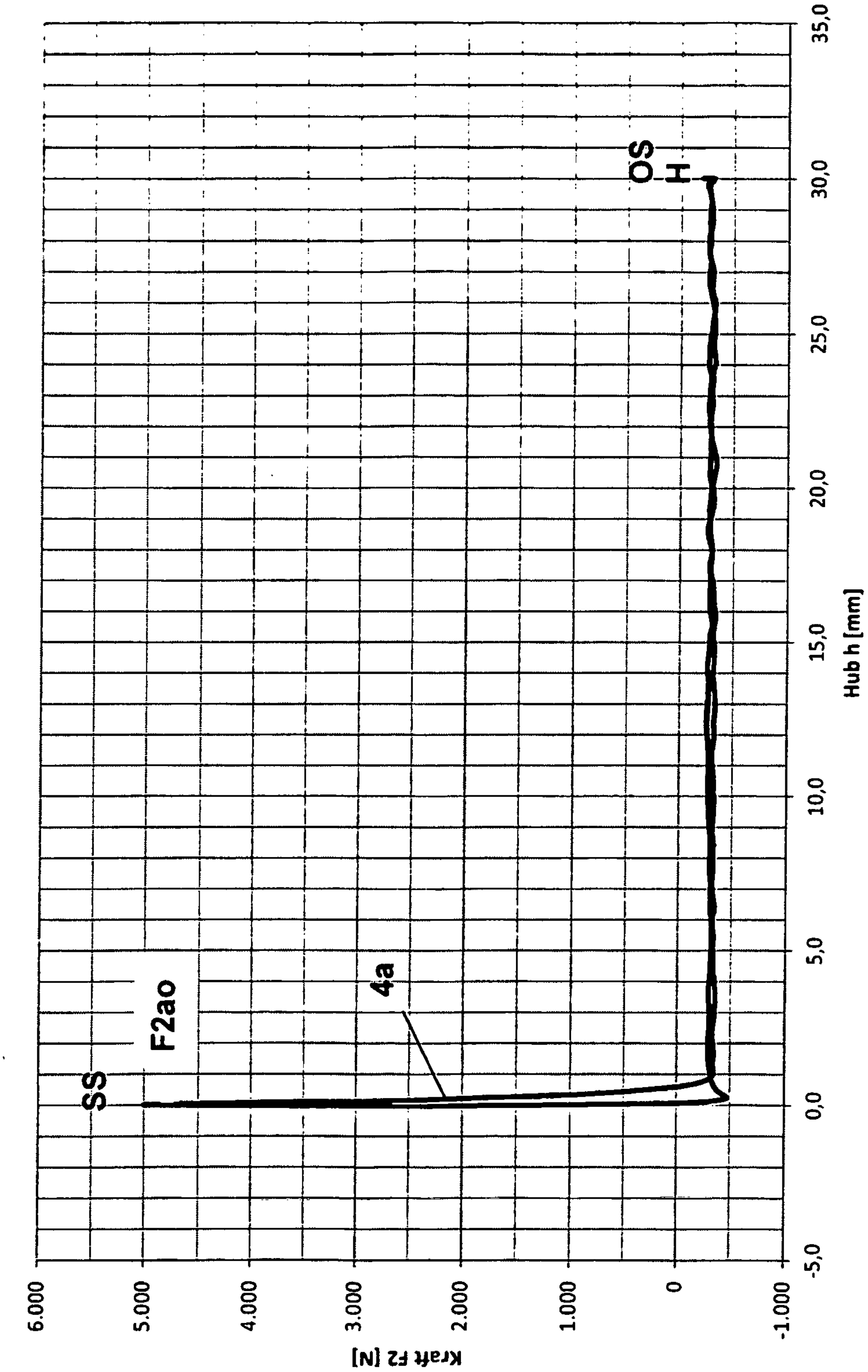
SS
F2ao
4a
OS
H
-5,0
0,0
5,0
10,0
15,0
20,0
25,0
30,0
35,0
Hub h [mm]
6.000
5.000
4.000
3.000
2.000
1.000
0
-1.000
Kraft F2 [N]

Figur 6

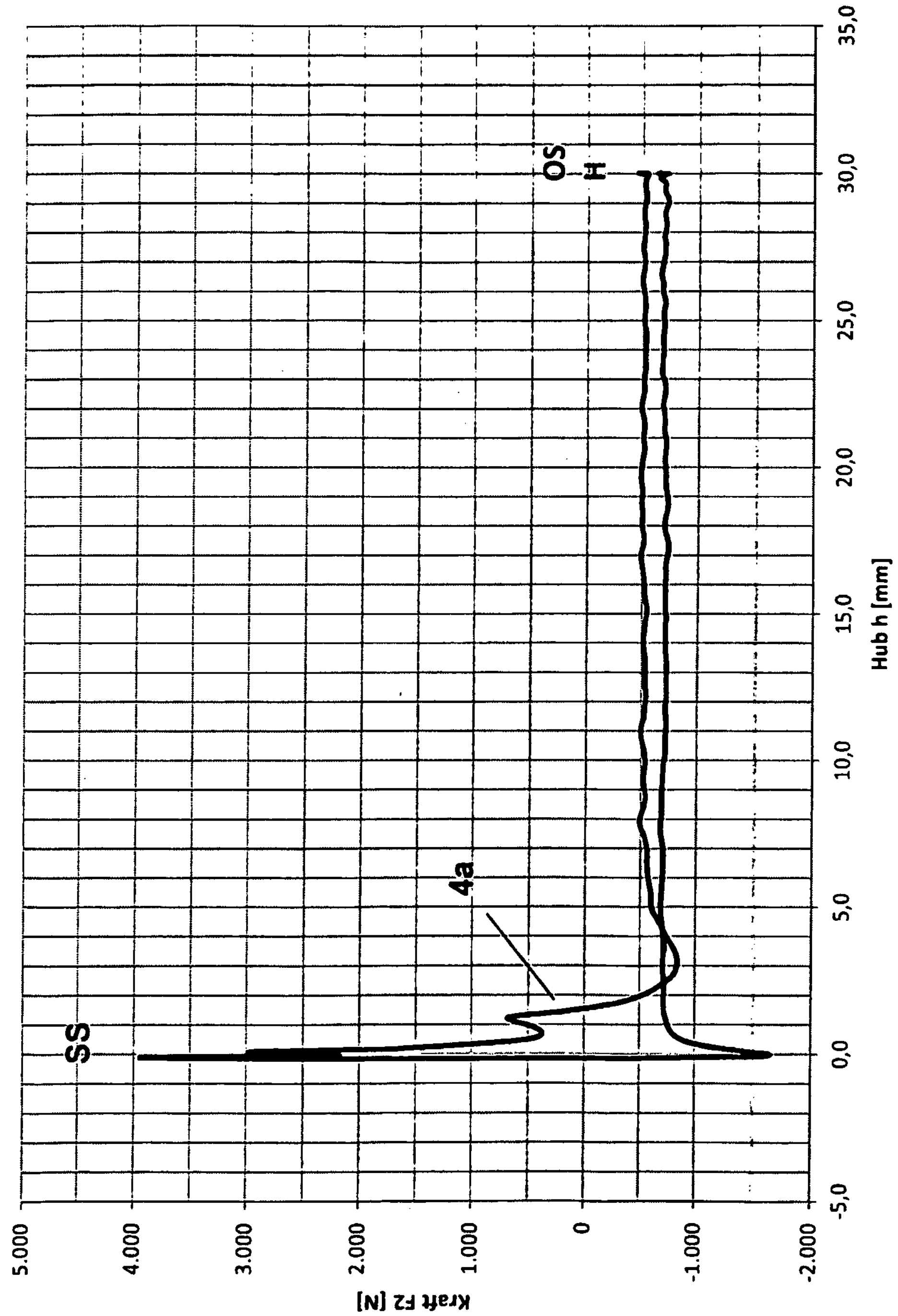
SS
OS
H
4a
5.000
4.000
3.000
2.000
1.000
0
-1.000
-2.000
Kraft F2 [N]
-5,0
0,0
5,0
10,0
15,0
20,0
25,0
30,0
35,0
Hub h [mm]

Figur 6a

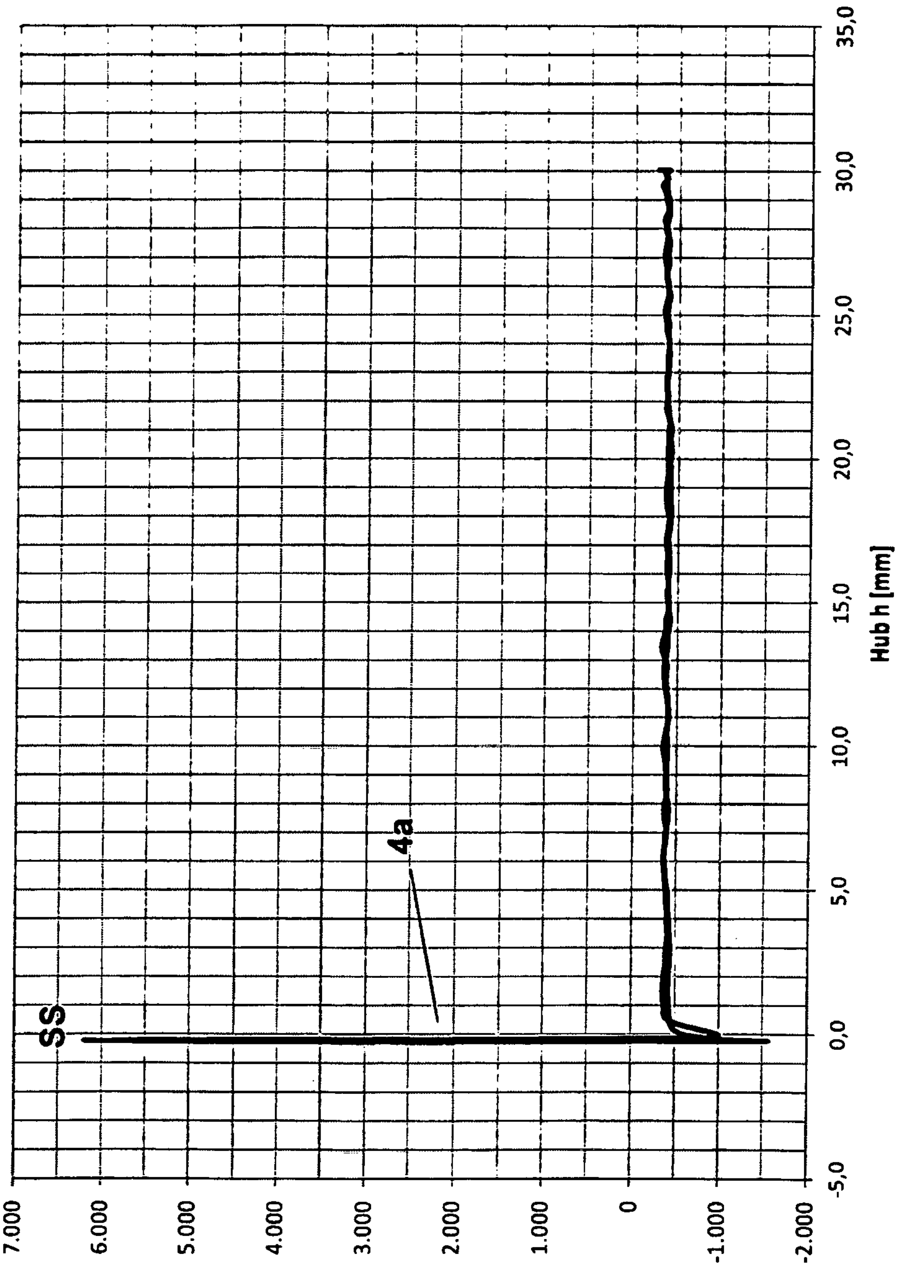

SS
4a
Kraft F2 [N]
7.000
6.000
5.000
4.000
3.000
2.000
1.000
0
-1.000
-2.000
Hub h [mm]
-5,0
0,0
5,0
10,0
15,0
20,0
25,0
30,0
35,0


Figur 6b

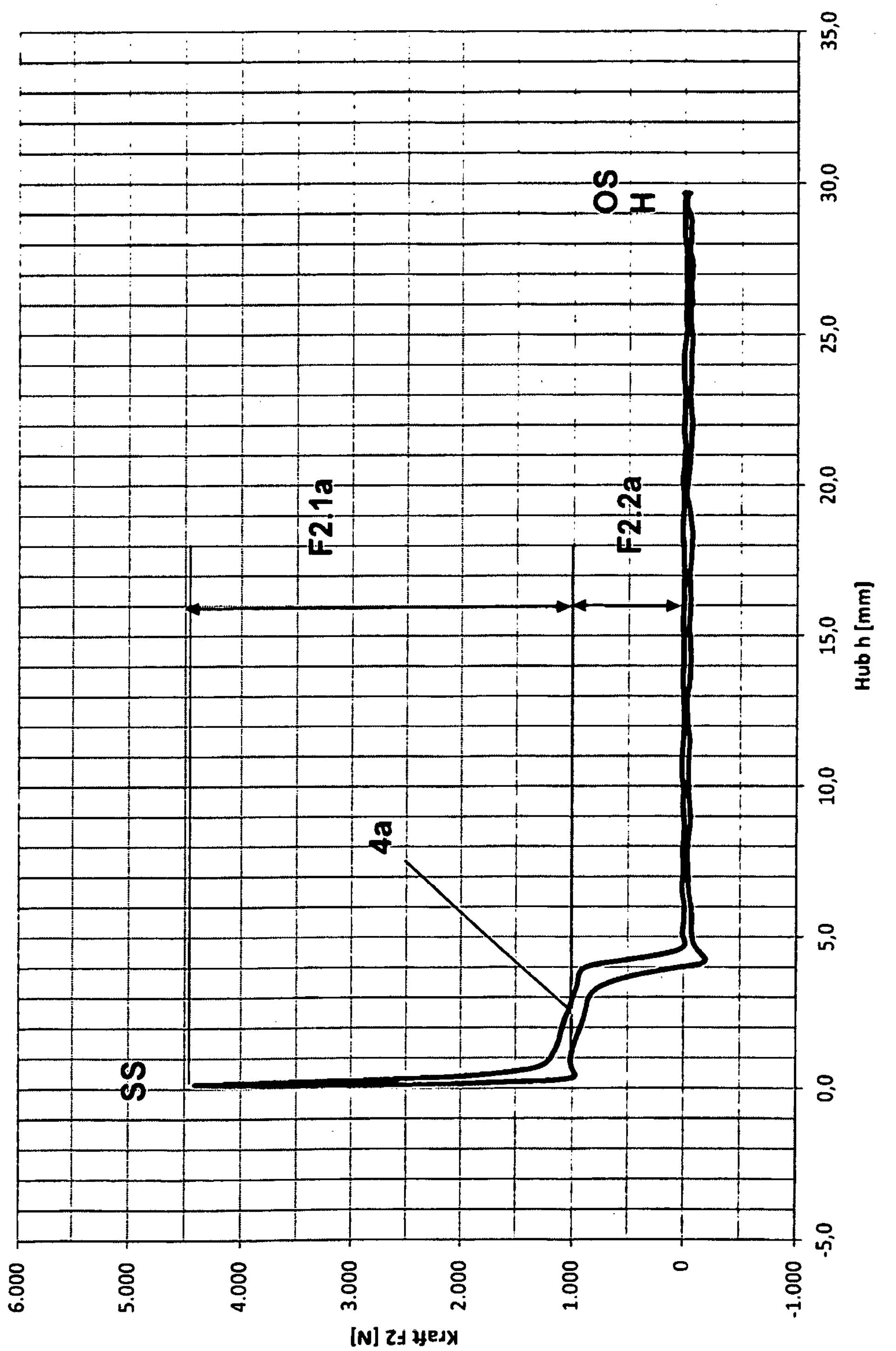
SS
OS
H
F2.1a
F2.2a
4a
6.000
5.000
4.000
3.000
2.000
1.000
0
-1.000
Kraft F2 [N]
-5,0
0,0
5,0
10,0
15,0
20,0
25,0
30,0
35,0
Hub h [mm]

Figur 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 29811115 U1
- WO 9630684 A1
- US 4882937 A
- EP 1529176 B1
- DE 19608792 C2
- WO 02093058 A1
- EP 1387975 B1